(19) Europäisches Patentamt
European Patent Office
Office européen des brevets

(11) Numéro de publication: **0 520 883 B1**

(12) **FASCICULE DE BREVET EUROPEEN**

(45) Date de publication de fascicule du brevet: **12.07.95**

(51) Int. Cl.[6]: **C07D 239/42**, C07D 239/46, C07D 401/12, C07D 405/12, C07D 401/14, A61K 31/505

(21) Numéro de dépôt: **92401773.4**

(22) Date de dépôt: **24.06.92**

(54) **Dérivés de 2-aminopyrimidine-4-carboxamide, leur préparation et leur application en thérapeutique.**

(30) Priorité: **27.06.91 FR 9107939**

(43) Date de publication de la demande:
**30.12.92 Bulletin 92/53**

(45) Mention de la délivrance du brevet:
**12.07.95 Bulletin 95/28**

(84) Etats contractants désignés:
**AT BE CH DE DK ES FR GB GR IT LI LU MC NL PT SE**

(56) Documents cités:
**DE-A- 2 143 730**

(73) Titulaire: **SYNTHELABO**
**22, Avenue Galilée**
**F-92350 Le Plessis Robinson (FR)**

(72) Inventeur: **George, Pascal**
**19, rue des Ouatre Vents**
**F-78730 St Arnoult en Yvelines (FR)**
Inventeur: **Manoury, Philippe**
**L'Orée de Verrières,**
**38, avenue des Vaupépins**
**91370 Verrières le Buisson (FR)**
Inventeur: **Marabout, Benoit**
**6, rue Georges Ritz**
**91300 Massy (FR)**
Inventeur: **Froissant, Jacques**
**Cidex 981 Brevainville**
**41160 Morée (FR)**
Inventeur: **Merly, Jean-Pierre**
**11, avenue Jules Guesde**
**92330 Sceaux (FR)**

(74) Mandataire: **Ludwig, Jacques et al**
**SYNTHELABO,**
**Service Brevets,**
**B.P. 72**
**F-92352 Le Plessis Robinson Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 520 883 B1

## Description

La présente invention a pour objet des dérivés de 2-aminopyrimidine-4-carboxamide, leur préparation et leur application en thérapeutique.

Les composés de l'invention répondent à la formule générale (I)

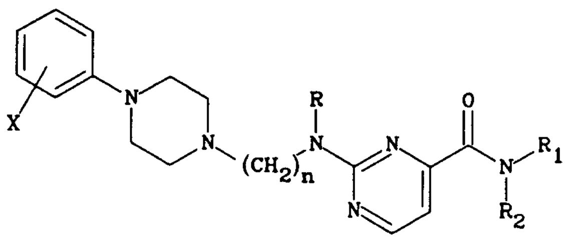

(I)

dans laquelle

- n représente le nombre 2 ou 3,
- X représente un ou plusieurs atomes ou groupes choisis parmi les suivants : hydrogène, fluor, chlore, méthoxy,
- R représente un atome d'hydrogène ou un groupe méthyle,
- $R_1$ représente un atome d'hydrogène ou un groupe méthyle,
- $R_2$ représente
  - un groupe alkyle en $C_1$-$C_6$,
  - un groupe hydroxyalkyle en $C_2$-$C_3$,
  - un groupe (hydroxy) (méthoxy)alkyle en $C_2$-$C_3$,
  - un groupe diméthoxyalkyle en $C_2$-$C_3$,
  - un groupe 2-(aminosulfonyl)éthyle,
  - un groupe 2-(méthylsulfonyl)éthyle,
  - un groupe 2-(méthylsulfonylamino)éthyle,
  - un groupe de formule générale $-CH_2-CO-NY_1Y_2$ (dans laquelle $Y_1$ et $Y_2$, indépendamment l'un de l'autre, représentent chacun un atome d'hydrogène ou un groupe alkyle en $C_1$-$C_6$),
  - un groupe de formule générale $-(CH_2)_2-Ar$ (dans laquelle Ar représente un groupe phényle éventuellement substitué par un ou plusieurs groupes méthoxy ou aminosulfonyle),
  - un groupe de formule générale

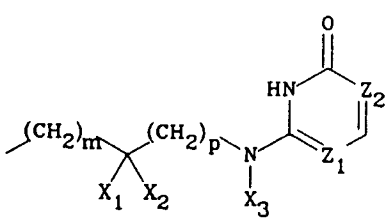

ou (tautomère)

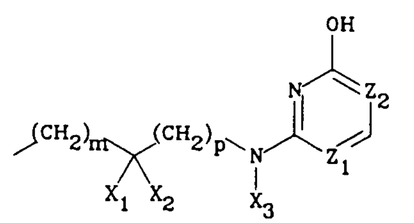

dans lesquelles m représente le nombre 0 ou 1, p représente le nombre 1 ou 2, l'un des symboles $Z_1$ et $Z_2$ représente un groupe CH et l'autre représente un atome d'azote et, soit $X_1$, $X_2$ et $X_3$ représentent chacun un atome d'hydrogène, soit $X_1$ et $X_2$ représentent chacun un groupe méthyle et $X_3$ représente un atome d'hydrogène, soit $X_1$ représente un atome d'hydrogène et $X_2$ et $X_3$ forment ensemble une chaîne de formule générale $-(CH_2)_{4-p}$ (p étant tel que défini ci-dessus), ou

  - un groupe de formule générale

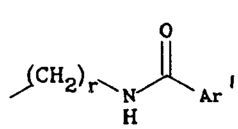

(dans laquelle r représente le nombre 2 ou 3 et Ar' représente soit un groupe phényle éventuellement substitué par un ou plusieurs groupes chloro, méthoxy ou amino, soit un groupe furan-2-yle, soit un groupe tétrahydrofuran-2-yle, soit un groupe pyridin-3-yle),

ou bien encore $R_1$ et $R_2$ forment, avec l'atome d'azote qui les porte,

- un groupe de formule générale

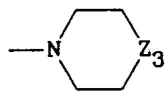

dans laquelle $Z_3$ représente un atome d'oxygène ou de soufre, ou un groupe sulfonyle, ou un groupe de formule générale N-$R_4$ dans laquelle $R_4$ représente un atome d'hydrogène, un groupe méthyle, un groupe acétyle, un groupe tert.-butyloxycarbonyle, un groupe phényloxycarbonyle ou un groupe de formule générale

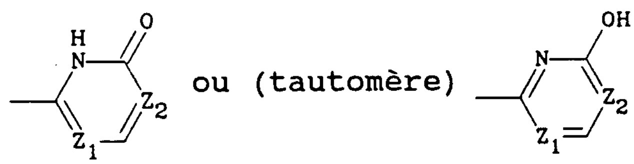

dans laquelle $Z_1$ et $Z_2$ sont tels que définis ci-dessus, ou

- un groupe de formule générale

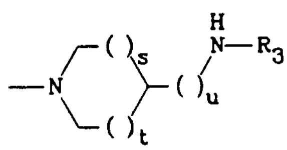

dans laquelle s représente soit le nombre 0, auquel cas t représente le nombre 2, soit le nombre 1, auquel cas t représente le nombre 1, u représente le nombre 0 ou 1 et $R_3$ représente un atome d'hydrogène, un groupe tert.-butyloxycarbonyle, un groupe benzyloxycarbonyle ou un groupe de formule générale

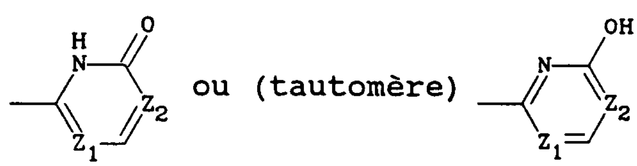

dans laquelle $Z_1$ et $Z_2$ sont tels que définis ci-dessus.

Les composés de l'invention peuvent se présenter à l'état de bases libres ou de sels d'addition à des acides pharmaceutiquement acceptables.

Conformément à l'invention, on peut préparer les composés de formule générale (I) selon un procédé illustré par le schéma de la page suivante.

On transforme un amide de formule générale (II), dans laquelle n, X et R sont tels que définis ci-dessus, en ester de formule générale (III) dans laquelle R' représente un groupe alkyle en $C_1$-$C_4$, par réaction avec un alcool aliphatique, par exemple le méthanol, en présence d'un acide, par exemple l'acide chlorhydrique gazeux, à une température de 0 à 60 °C, puis on fait réagir l'ester ainsi obtenu avec une amine de formule générale (IV), dans laquelle $R_1$ et $R_2$ sont tels que définis ci-dessus.

Lorsque l'amine de formule générale (IV) est primaire ($R_1$ = H), on effectue la réaction dans un alcool aliphatique, par exemple le méthanol ou le n-butanol, à une température de 0 à 100 °C.

Lorsque l'amine de formule générale (IV) est secondaire ($R_1$ = $CH_3$), on prépare préalablement l'amidure de diméthylaluminium correspondant au moyen de triméthylaluminium, dans un solvant inerte tel que l'hexane,

le toluène ou le dichlorométhane, et c'est l'amidure ainsi obtenu qu'on fait réagir avec l'ester de formule générale (III), dans le dichlorométhane à une température de 0 à 40 ° C.

**Schéma**

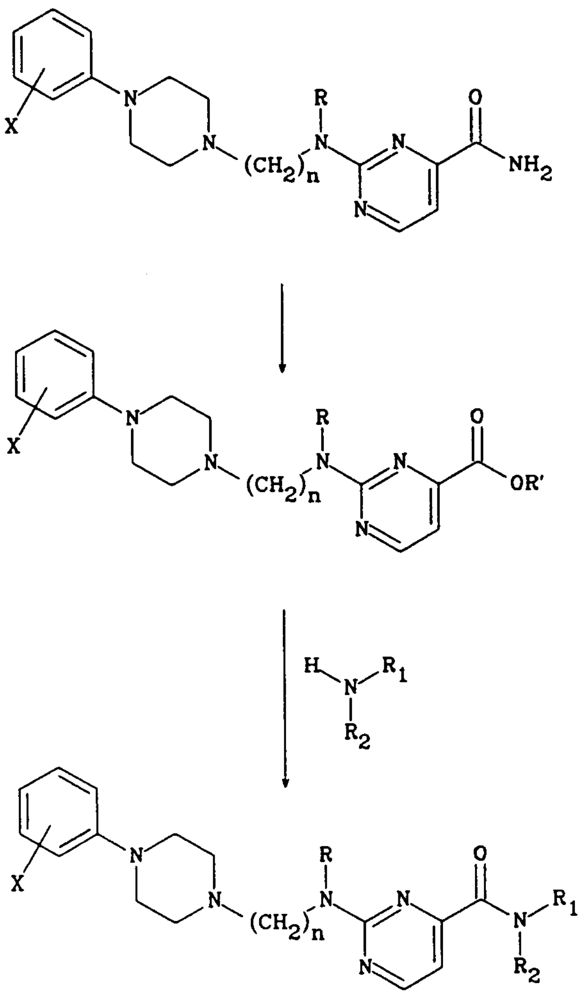

(II)

(III)

(IV)

(I)

Les composés de départ de formule générale (II) peuvent être préparés par des méthodes analogues à celles décrites dans la demande de brevet EP-0435749.

Les amines de formule générale (IV) dans laquelle $R_1$ représente un atome d'hydrogène et $R_2$ représente un groupe de formule générale

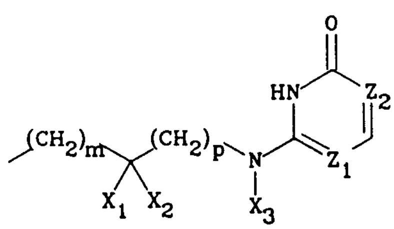

peuvent être préparées par des méthodes analogues à celles décrites dans *Biochem. Biophys. Res. Comm.* (1990) **170**(1), 243 et dans la demande de brevet EP-0373891.

Les amines de formule générale (IV) dans laquelle $R_1$ représente un atome d'hydrogène et $R_2$ représente un groupe de formule générale

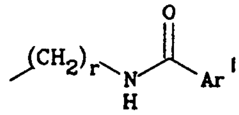

peuvent être préparées par des méthodes analogues aux méthodes mentionnées ci-dessus.

Les amines de formule générale (IV) dans laquelle $R_1$ représente un atome d'hydrogène et $R_2$ représente un groupe de formule générale $-CH_2-CO-NY_1Y_2$ peuvent être préparées par des méthodes analogues à celles décrites dans les demandes de brevets EP-0062161, EP-0227410 et EP-0316179.

Enfin, dans le cas des amines de formule générale (IV) dans laquelle $R_1$ et $R_2$ représentent ensemble un groupe de formule générale

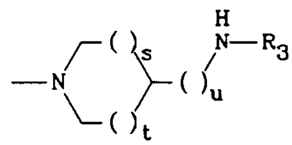

on utilise une telle diamine protégée, dans la formule de laquelle $R_3$ représente un groupement protecteur tel qu'un groupe benzyloxycarbonyle ou tert.-butyloxycarbonyle. De telles diamines protégées peuvent être préparées par des méthodes analogues à celles décrites à propos de la synthèse du pipéridine-4-carbamate de 1,1-diméthyléthyle, dans les demandes de brevets DE-2831431, EP-0410278 et EP-0417698. Si on le désire, on déprotège le composé obtenu selon une méthode connue, par exemple par l'acide trifluoroacétique dans le dichlorométhane (dans le cas d'un groupement tert.-butyloxycarbonyle), pour obtenir un composé dans la formule duquel $R_3$ représente l'hydrogène et, si on le désire, on fait réagir cette dernière avec la 2,3-dihydro-4-thioxo-pyrimidin-2(1*H*)-one ou la 2-méthylthiopyrimidin-4(1*H*)-one, selon une méthode analogue à celle décrite dans la demande de brevet EP-0373891.

Il va de soi que les diverses modifications qui viennent d'être décrites à propos de l'amine protégée de formule générale (IV) peuvent être effectuées directement sur ladite amine, comme décrit ci dessus, mais aussi sur le composé de formule générale (I), après la réaction de l'amine protégée de formule générale (IV) avec l'ester de formule générale (III).

Les exemples suivants illustrent la préparation de quelques composés selon l'invention.

Les micronalyses élémentaires et les spectres IR et RMN confirment les structures des produits obtenus.

Les numéros de composés indiqués entre parenthèses dans les titres des exemples correspondent à ceux du tableau donné plus loin.

Example 1 (Composé N° 1)

2-[[3-[4-(3-Chlorophényl)pipérazin-1-yl]propyl]amino]-*N*-méthylpyrimidine-4-carboxamide, chlorhydrate.

1.1. 2-[[3-[4-(3-Chlorophényl)pipérazin-1-yl]propyl]amino]pyrimidine-4-carboxylate de méthyle.

Dans un ballon de 1 l, on introduit 20,76 g (55,4 mmoles) de 2-[[3-[4-(3-chlorophényl)pipérazin-1-yl]-propyl]amino]pyrimidine-4-carboxamide et 600 ml de méthanol puis on fait passer un courant d'acide chlorhydrique gazeux pendant quelques minutes et on chauffe, à la température du reflux du méthanol, pendant 1,5 heure.

On évapore le solvant sous pression réduite, on ajoute au résidu 200 ml de dichlorométhane et on refroidit à 0 °C. On alcalinise le mélange avec une solution aqueuse saturée d'hydrogénocarbonate de sodium, on décante et sèche la phase organique sur sulfate de sodium, on filtre puis évapore le solvant sous pression réduite.

Après chromatographie sur colonne de silice (éluant : mélange acétate d'éthyle/méthanol, 100/0 à 95/5) puis recristallisation dans du cyclohexane, on isole 16,16 g (41,5 mmoles) de composé.

Point de fusion : 100,5-101 °C.

1.2. 2-[[3-[4-(3-Chlorophényl)pipérazin-1-yl]propyl]amino]-*N*-méthylpyrimidine-4-carboxamide, chlorhydrate.

Dans un ballon de 0,5 l, on introduit 4,5 g (11,5 mmoles) de 2-[[3-[4-(3-chlorophényl)pipérazin-1-yl]-propyl]amino]pyrimidine-4-carboxylate de méthyle et 200 ml de méthanol, puis on sature la solution de méthylamine gazeuse. On agite le mélange réactionnel à la température ambiante en saturant plusieurs fois en méthylamine gazeuse. On évapore le solvant sous pression réduite. On obtient 4,05 g (10,4 mmoles) de base à laquelle on ajoute 104 ml d'acide chlorhydrique 0,1N dans du propan-2-ol. On évapore le solvant et on fait recristalliser le produit dans de la butan-2-one. On obtient 3,8 g de composé.
Point de fusion : 166-168 ° C.

Exemple 2 (Composé N ° 60)

2-[[3-[4-(3-Chlorophényl)pipérazin-1-yl]propyl]amino]-*N*,*N*-diméthylpyrimidine-4-carboxamide, dichlorhydrate.

Dans un ballon de 0,5 l, on introduit 4 g (10,2 mmoles) de 2-[[3-[4-(3-chlorophényl)pipérazin-1-yl]-propyl]amino]pyrimidine-4-carboxylate de méthyle et 200 ml de méthanol, puis on sature la solution avec de la diméthylamine gazeuse. On agite le mélange réactionnel pendant trois jours à la température ambiante en saturant plusieurs fois en diméthylamine gazeuse. On évapore le solvant sous pression réduite et on obtient, après chromatographie sur silice (éluant : mélange acétate d'éthyle/méthanol 100/0 à 90/10), 2,24 g (5,55 mmoles) du composé sous forme de base.
A 1,69 g de base, on ajoute 80 ml d'acide chlorhydrique 0,1N dans du propan-2-nol. On évapore le solvant et on fait recristalliser le produit dans de l'acétone. On obtient 1,94 g (4,08 mmoles) de composé.
Point de fusion : 194-198 ° C.

Exemple 3 (Composé N ° 69)

1-[2-[[3-[4-(5-Chloro-2-méthoxyphényl)pipérazin-1-yl]propyl]amino]pyrimidin-4-ylcarbonyl]-4-méthylpipérazine, dichlorhydrate.

3.1. 2-[[3-[4-(5-Chloro-2-méthoxyphényl)pipérazin-1-yl]propyl]amino]pyrimidine-4-carboxylate de méthyle.

Dans un ballon de 0,5 l, on introduit 7,8 g (19,2 mmoles) de 2-[[3-[4-(5-chloro-2-méthoxyphényl)-pipérazin-1-yl]propyl]amino]pyrimidine-4-carboxamide et 300 ml de méthanol, puis on fait passer un courant d'acide chlorhydrique gazeux pendant quelques minutes et on chauffe le mélange à la température du reflux du méthanol pendant 1,75 heures. On évapore le solvant sous pression réduite, on ajoute au résidu 200 ml de dichlorométhane puis on refroidit à 0 ° C. On alcalinise le mélange avec une solution aqueuse saturée d'hydrogénocarbonate de sodium, on sèche la phase organique sur sulfate de sodium, on filtre puis évapore le solvant sous pression réduite.
Après chromatographie sur colonne de silice (éluant : mélange dichlorométhane/méthanol, 100/0 à 90/10) puis recristallisation dans du cyclohexane, on isole 5,84 g (13,9 mmoles) d'ester.
Point de fusion : 118,5-119 ° C.

3.2. 1-[2-[[3-[4-(5-Chloro-2-méthoxyphényl)pipérazin-1-yl]propyl]amino]pyrimidin-4-ylcarbonyl]-4-méthylpipérazine, dichlorhydrate.

Dans un ballon de 0,25 l, on introduit successivement 80 ml de dichlorométhane, 6,8 g d'une solution à 25 % de triméthylaluminium dans de l'hexane (23,6 mmoles) puis 2,96 g (29,5 mmoles) de 1-méthylpipérazine dans 10 ml de dichlorométhane. On agite le mélange réactionnel pendant 30 minutes à la température ambiante, on ajoute alors lentement 1,84 g (4,72 mmoles) de 2-[[3-[4-(5-chloro-2-méthoxyphényl)pipérazin-1-yl] propyl]amino]pyrimidine-4-carboxylate de méthyle dans 10 ml de dichlorométhane. On agite le mélange réactionnel pendant 30 minutes à la température ambiante puis pendant 3,5 heures à la température de reflux.
On refroidit le mélange réactionnel à 0 ° C avec un mélange eau/sel/glace, on hydrolyse par quelques ml d'eau, on laisse agiter 1 heure à la température ambiante, on filtre, on ajoute de l'eau et on extrait au dichlorométhane (3×150 ml). On évapore le solvant sous pression réduite, on chromatographie l'huile obtenue sur colonne de gel de silice (éluant : mélange dichlorométhane/méthanol, 100/0 à 80/20) pour obtenir 1,29 g de base.
A la base en solution dans 10 ml de dichlorométhane on ajoute 56 ml d'acide chlorhydrique 0,1N dans du

propan-2-nol, on évapore sous pression réduite et on fait cristalliser le résidu dans de l'acétone. On obtient 1 g (1,88 mmoles) de dichlorhydrate.
Point de fusion : 253-256 °C.

Exemple 4 (Composé N° 30)

2-[[3-[4-(5-Fluoro-2-méthoxyphényl)pipérazin-1-yl]propyl]amino]-*N*-[3-[(2-oxo-1,2-dihydropyrimidin-4-yl)-amino]propyl]pyrimidine-4-carboxamide.

4.1. 2-[[3-[4-(5-Fluoro-2-méthoxyphényl)pipérazin-1-yl]propyl]amino]pyrimidine-4-carboxylate de méthyle.

Dans un ballon de 0,5 l, on introduit 3,0 g (7,72 mmoles) de 2-((3-(4-(5-fluoro-2-méthoxyphényl)-pipérazin-1-yl]propyl]amino]-pyrimidine-4-carboxamide et 250 ml de méthanol, puis on fait passer un courant d'acide chlorhydrique gazeux pendant quelques minutes et on chauffe le mélange à la température du reflux du méthanol pendant 1,5 heure. On évapore le solvant sous pression réduite, on ajoute au résidu 150 ml de dichlorométhane puis on refroidit à 0 °C. On alcalinise le mélange avec une solution aqueuse saturée d'hydrogénocarbonate de sodium, on décante la phase organique puis on la sèche sur sulfate de sodium, et on filtre puis évapore le solvant sous pression réduite.
Après chromatographie sur colonne de silice (éluant : mélange dichlorométhane/méthanol, 99/1 à 95/5), puis recristallisation dans du cyclohexane, on isole 2,6 g (6,44 mmoles) d'ester.
Point de fusion : 102-103 °C.

4.2. 2-[(3-[4-(5-Fluoro-2-méthoxyphényl)pipérazin-1-yl]propyl]amino]-*N*-[3-[(2-oxo-1,2 dihydropyrimidin-4-yl)-amino]propyl]pyrimidine-4-carboxamide.

Dans un ballon de 0,1 l, on introduit 1 g (2,48 mmoles) de 2[[3-[4-(5-fluoro-2-méthoxyphényl)pipérazin-1-yl]propyl]amino]pyrimidine-4-carboxylate de méthyle et 0,55 g (3,27 mmoles) de 4-[(3-aminopropyl)-amino]pyrimidin-2(1*H*)-one dans 15 ml de propan-2-ol, et on chauffe le mélange à la température de reflux du solvant pendant 14 heures.
Le produit précipite dans le milieu réactionnel ; on le collecte par filtration puis on le recristallise dans un mélange de dichlorométhane et de méthanol. On obtient 0,74 g (1,37 mmoles) de solide blanc.
Point de fusion : 219-221 °C.

Exemple 5 (Composé N° 23)

2-[[3-[4-(5-Chloro-2-méthoxyphényl)pipérazin-1-yl]propyl]amino]-*N*-[2-[(4-oxo-1,4-dihydropyrimidin-2-ylamino]éthyl]pyrimidine-4-carboxamide.

Dans un ballon de 25 ml, on introduit 1,89 g (4,5 mmoles) de 2-[[3-[4-(5-chloro-2-méthoxyphényl)-pipérazin-1-yl]propyl]amino]pyrimidine-4-carboxylate de méthyle et 0,77 g (5 mmoles) de 2-[(2-aminoéthyl)-amino]pyrimidin-4(1*H*)-one dans 5 ml de n-butanol. On chauffe le mélange à la température du reflux du solvant pendant 15 heures, puis on évapore le solvant sous pression réduite.
On purifie le produit brut par chromatographie sur colonne de gel de silice (éluant : mélange dichlorométhane/méthanol, 95/5 à 80/20), puis on recristallise le produit obtenu dans un mélange de dichlorométhane et d'acétate d'éthyle, pour obtenir 1,04 g (1,92 mmoles) de solide blanc.
Point de fusion : 118-120 °C.

Exemple 6 (Composé N° 35)

2-[[2-[4-(5-Chloro-2-méthoxyphényl)pipérazin-1-yl]éthyl]amino]-*N*-[3-[(4-oxo-1,4-dihydropyrimidin-2-yl)-amino]propyl]pyrimidine-4-carboxamide.

6.1. 2-[[2-[4-(5-Chloro-2-méthoxyphényl)pipérazin-1-yl]éthyl]amino]pyrimidine-4-carboxylate de méthyle.

Dans un ballon de 1 litre, on introduit 6,6 g (16,88 mmoles) de 2-[[2-[4-(5-chloro-2-méthoxyphényl)-pipérazin-1-yl]éthyl]amino]pyrimidine-4-carboxamide et 600 ml de méthanol, puis on fait passer un courant d'acide chlorhydrique gazeux pendant quelques minutes et on chauffe le mélange à la température du reflux du méthanol pendant 3 heures. On évapore le solvant sous pression réduite, on ajoute au résidu 100

ml de dichlorométhane puis on refroidit à 0 °C. On alcalinise le mélange avec une solution aqueuse saturée d'hydrogénocarbonate de sodium, on décante la phase organique, on la sèche sur sulfate de magnésium, on la filtre, puis évapore le solvant sous pression réduite.
Après chromatographie sur colonne de gel de silice (éluant dichlorométhane/méthanol, 100/0 à 95/5) puis recristallisation dans du cyclohexane, on isole 4,5 g (11,09 mmoles) d'ester.
Point de fusion : 127-129 °C.

6.2. 2-[[2-[4-(5-Chloro-2-méthoxyphényl)pipérazin-1-yl]éthyl]amino]-*N*-[3-[(4-oxo-1,4-dihydropyrimidin-2-yl)-amino]propyl]pyrimidine-4-carboxamide.

Dans un ballon de 100 ml, on introduit 3,0 g ( 7,4 mmoles) de 2-[[2-[4-(5-chloro-2-méthoxyphényl)-pipérazin-1-yl]éthyl]amino]pyrimidine-4-carboxylate de méthyle et 1,5 g (8,9 mmoles) de 2-[(3-aminopropyl)-amino]pyrimidin-4(1*H*)-one dans 20 ml de n-butanol. On chauffe le mélange à la température du reflux du solvant pendant 20 heures, puis on évapore le solvant sous pression réduite.
On purifie le produit brut par chromatographie sur colonne de gel de silice (éluant : mélange dichlorométhane/méthanol, 98/2 à 80/20), puis on recristallise le produit obtenu dans un mélange dichlorométhane/acétate d'éthyle pour obtenir 1,7 g (3,14 mmoles) de solide blanc.
Point de fusion : 100-102 °C.

Exemple 7 (Composé N° 79)

1-[2-[[2-[4-(5-Chloro-2-méthoxyphényl)pipérazin-1-yl]éthyl]amino]pyrimidin-4-ylcarbonyl]pipéridine-4-carbamate de 1,1-diméthyléthyle, chlorhydrate.

Dans un ballon de 0,5 l, on introduit successivement 150 ml de dichlorométhane, 10,1 g d'une solution à 25% de triméthylaluminium dans de l'hexane puis 7,0 g (35 mmoles) de (pipéridin-4-yl)carbamate de 1,1-diméthyléthyle dans 10 ml de dichlorométhane. On agite le mélange réactionnel pendant 30 minutes à la température ambiante, puis on ajoute lentement 3,9 g (9,6 mmoles) de 2-[[2-[4-(5-chloro-2-méthoxyphényl)-pipérazin-1-yl]éthyl]amino]pyrimidine-4-carboxylate de méthyle dans 20 ml de dichlorométhane. On agite le mélange réactionnel pendant 30 minutes à la température ambiante et 3 heures à la température du reflux du dichlorométhane, puis on refroidit le mélange réactionnel à 0 °C avec un mélange eau/sel/glace. On hydrolyse par quelques ml d'eau, on laisse agiter 1 heure à la température ambiante, on filtre et évapore les solvants sous pression réduit. On purifie l'huile obtenue par chromatographie sur gel de silice (éluant : mélange dichlorométhane/méthanol, 98/2 à 90/10) pour obtenir 4,92 g (8,57 mmoles) de base.
A la base en solution dans 10 ml de dichlorométhane, on ajoute 82,5 ml d'acide chlorhydrique 0,1N dans le propan-2-ol, on évapore sous pression réduite et on fait recristalliser le produit dans l'acétate d'éthyle. On obtient 4,61 g (7,55 mmoles) de composé.
Point de fusion : 194-197 °C.

Exemple 8 (Composé N° 81)

1-[2-[[2-[4-(5-Chloro-2-méthoxyphényl)pipérazin-1-yl]éthyl]amino]pyrimidin-4-ylcarbonyl]pipéridin-4-amine.

Dans un ballon de 0,5 l, on place 3 g (4,9 mmoles) de chlorhydrate de 1-[2-[[2-[4-(5-chloro-2-méthoxyphényl)pipérazin-1-yl ]éthyl]amino]pyrimidin-4-ylcarbonyl]pipéridine-4-carbamate de 1,1-diméthyléthyle en solution dans 20 ml d'eau puis, goutte à goutte, 10 ml d'acide chlorhydrique concentré. On agite 5 minutes à température ambiante, puis on refroidit à 0 °C avec un mélange glace/sel/eau. On ajoute au milieu réactionnel de la soude aqueuse à 30% jusqu'à pH≥8, puis on extrait au dichlorométhane, on sèche sur sulfate de magnésium, on filtre et on évapore le solvant sous pression réduite, pour obtenir 2,3 g (4,9 mmoles) de solide amorphe.

Exemple 9 (Composé N° 85)

2-[[1-[2-[[2-[4-(5-Chloro-2-méthoxyphényl)pipérazin-1-yl]éthyl]amino]pyrimidin-4-ylcarbonyl]pipéridin-4-yl]-amino]pyrimidin-4(1*H*)-one.

Dans un ballon de 0,5 l, on introduit 2,3 g (4,9 mole) de 1[2-[[2-[4-(5-chloro-2-méthoxyphényl)pipérazin-1-yl]éthyl]amino]pyrimidin-4-ylcarbonyl]pipéridin-4-amine, 0,9 g (6,3 mmoles) de 2-méthylthiopyrimidin-4-

(1*H*)-one en solution dans 40 ml de m-xylène et on chauffe au reflux du m-xylène pendant 14 heures. On refroidit le milieu réactionnel à température ambiante, puis on évapore le solvant sous pression réduite. On purifie le produit brut par chromatographie sur colonne de gel de silice (éluant : dichlorométhane/méthanol, 98/2 à 85/15) pour obtenir 1,56 g (2,74 mmoles) de composé.
Point de fusion : 114-117 °C.

Exemple 10 (Composé N° 59)

2-[[2-[4-(5-Chloro-2-méthoxyphényl)pipérazin-1-yl]éthyl]amino]-*N*-[2-[(furan-2-ylcarnonyl)amino]éthyl]-pyrimidine-4-carboxamide, oxalate.

Dans un ballon de 25 ml on introduit 1,22 g (3 mmoles) de 2-([2-[4-(5-chloro-2-méthoxyphényl)-pipérazin-1-yl]éthyl]amino]pyrimidine-4-carboxylate de méthyle et 0,7 g (4,5 mmoles) de *N*-(furan-2-ylcarbonyl)éthanediamine en solution dans un mélange 3/1 de propan-2-ol/méthanol, puis on chauffe le milieu réactionnel au reflux pendant 7,5 heures.
On refroidit à température ambiante, on évapore les solvants sous pression réduite puis on chromatographie l'huile obtenue sur gel de silice (éluant : dichlorométhane/méthanol, 100/0 à 95/5) pour obtenir 1,58 g de base.

On dissout la base dans 50 ml d'éthanol et on ajoute 0,27 g (3 mmoles) d'acide oxalique. On évapore sous presion réduite puis on recristallise dans un mélange propan-2-ol/acétate d'éthyle pour obtenir 1,5 g (2,43 mmoles) de composé.
Point de fusion : 128-132 °C.

Exemple 11 (Composé N° 17)

[[2-[[3-[4-(5-Chloro-2-méthoxyphényl)pipérazin-1-yl]propyl]amino]pyrimidin-4-ylcarbonyl]amino]-*N*-propylacétamide.

Dans un ballon de 0,1 l, on introduit 2,4 g (5,7 mmoles) de 2-[[3-[4-(5-chloro-2-méthoxyphényl)-pipérazin-1-yl]propyl]amino]pyrimidine-4-carboxylate de méthyle, 2,2 g (14,4 mmoles) de chlorhydrate de *N*-propylglycinamide en solution dans un mélange de 30 ml de propan-2-ol et 30 ml de 1,2-dichloroéthane, puis on ajoute 2,1 ml (15 mmoles) de triéthylamine et on chauffe le mélange au reflux pendant 14 heures.
On refroidit à température ambiante, on ajoute 100 ml de dichlorométhane et 100 ml d'eau. On sépare la phase organique, on la sèche sur sulfate de sodium et on évapore les solvants sou pression réduite.
On purifie le produit brut par chromatographie sur gel de silice (éluant : dichlorométhane/méthanol, 98/2 à 90/10) et on recristallise le produit dans un mélange de dichlorométhane et d'acétonitrile, pour obtenir 1,8 g (3,57 mmoles) de composé.
Point de fusion : 143,5-144,5 °C.

Le tableau qui suit illustre les structures chimiques et les propriétés physiques de quelques composés selon l'invention.

Tableau

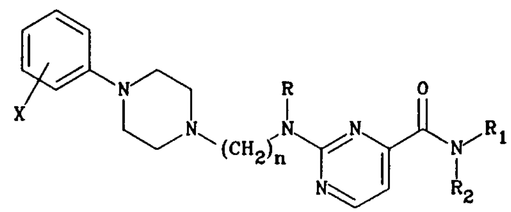

(I)

| N° | X | R | n | $-NR_1R_2$ | Sel ou base | F (°C) |
|---|---|---|---|---|---|---|
| 1 | 3-Cl | H | 3 | $-NH-CH_3$ | HCl | 166-168 |
| 2 | $2-OCH_3$, 5-Cl | H | 3 | $-NH-CH_3$ | HCl | 175,5-177,5 |
| 3 | $2-OCH_3$, 5-Cl | H | 3 | $-NH-(CH_2)_2-CH_3$ | $C_2H_2O_4$ | 173,5-175 |
| 4 | $2-OCH_3$, 5-Cl | H | 3 | $-NH-(CH_2)_5-CH_3$ | $C_2H_2O_4$ | 150-151,5 |
| 5 | $2-OCH_3$, 5-Cl | H | 3 | $-NH-(CH_2)_2-OH$ | base | 135-136 |
| 6 | 3-Cl | H | 3 | $-NH-(CH_2)_3-OH$ | $C_2H_2O_4$ | 157-159 |
| 7 | $2-OCH_3$, 5-Cl | H | 3 | $-NH-(CH_2)_3-OH$ | base | 94-95,5 |
| 8 | $2-OCH_3$, 5-Cl | H | 3 | $-NH-CH_2-CH(OH)-CH_2-OCH_3$ | base | 124,5-125,5 |
| 9 | $2-OCH_3$, 5-Cl | H | 3 | $-NH-CH_2-CH(OCH_3)-CH_2-OCH_3$ | $C_2H_2O_4$ | 148-150 |
| 10 | 3-Cl | H | 3 | $-NH-(CH_2)_2-SO_2-NH_2$ | 2HCl | 191-193 |
| 11 | $2-OCH_3$, 5-Cl | H | 3 | $-NH-(CH_2)_2-SO_2-NH_2$ | base | 165,5-167,5 |
| 12 | $2-OCH_3$, 5-Cl | H | 3 | $-NH-(CH_2)_2-SO_2-CH_3$ | base | 104,5-106,5 |
| 13 | 3-Cl | H | 3 | $-NH-(CH_2)_2-NH-SO_2-CH_3$ | 2HCl | 174-176 |
| 14 | $2-OCH_3$, 5-Cl | H | 3 | $-NH-(CH_2)_2-NH-SO_2-CH_3$ | base, $\frac{1}{2}H_2O$ | 127-129 |
| 15 | $2-OCH_3$, 5-Cl | H | 3 | $-NH-CH_2-CO-NH_2$ | HCl, $\frac{1}{2}H_2O$ | 195-197 |
| 16 | $2-OCH_3$, 5-Cl | H | 3 | $-NH-CH_2-CO-N(CH_3)_2$ | base | 153,5-154,5 |

| N° | X | R | n | $-NR_1R_2$ | Sel ou base | F (°C) |
|---|---|---|---|---|---|---|
| 17 | $2-OCH_3$, 5-Cl | H | 3 | $-NH-CH_2-CO-NH-(CH_2)_2-CH_3$ | base | 143,5-145,5 |
| 18 | $2-OCH_3$, 5-Cl | H | 3 | $-NH-CH_2-CO-NH-(CH_2)_5-CH_3$ | base | 122-124 |
| 19 | 3-Cl | H | 3 | 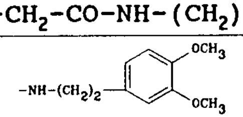 | 2HCl | 172,5-174 |
| 20 | $2-OCH_3$, 5-Cl | H | 3 | 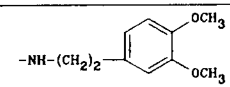 | 2HCl | 182-185 |
| 21 | $2-OCH_3$, 5-Cl | H | 3 | 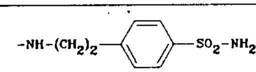 | 2HCl | 204-205 |
| 22 | $2-OCH_3$, 5-Cl | H | 2 | 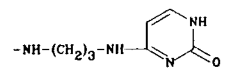 | base | 227-230 |
| 23 | $2-OCH_3$, 5-Cl | H | 3 | 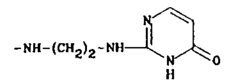 | base | 118-120 |
| 24 | $2-OCH_3$, 5-F | H | 3 | 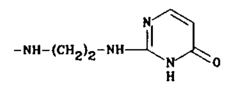 | base | 99-101 |
| 25 | $2-OCH_3$, 5-Cl | H | 3 | 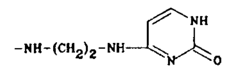 | base, $\frac{1}{2}H_2O$ | 229-232 |
| 26 | $2-OCH_3$, 5-F | H | 3 | 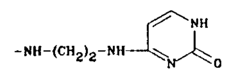 | base | 218,5-221 |
| 27 | $2-OCH_3$, 5-Cl | H | 3 | 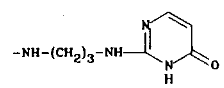 | base | 101-103 |
| 28 | $2-OCH_3$, 5-F | H | 3 | 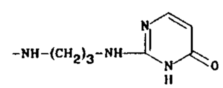 | base | 94-97 |

| N° | X | R | n | $-NR_1R_2$ | Sel ou base | F (°C) |
|---|---|---|---|---|---|---|
| 29 | $2-OCH_3$, 5-Cl | H | 3 | $-NH-(CH_2)_3-NH-$ 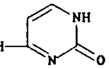 | base, $\frac{1}{2}H_2O$ | 218-219,5 |
| 30 | $2-OCH_3$, 5-F | H | 3 | $-NH-(CH_2)_3-NH-$ 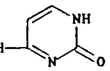 | base | 219-221 |
| 31 | 3-Cl | H | 2 | $-NH-(CH_2)_2-NH-$ 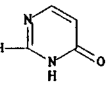 | base | 112-116 |
| 32 | $2-OCH_3$, 5-Cl | H | 2 | $-NH-(CH_2)_2-NH-$ 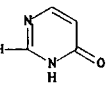 | base | 123-126 |
| 33 | 3-Cl | H | 2 | $-NH-(CH_2)_2-NH-$ 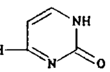 | base | 242-245 |
| 34 | 3-Cl | H | 2 | $-NH-(CH_2)_3-NH-$ 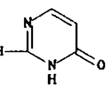 | base | 111-115 |
| 35 | $2-OCH_3$, 5-Cl | H | 2 | $-NH-(CH_2)_3-NH-$ 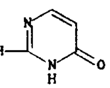 | base, $\frac{1}{2}H_2O$ | 100-102 |
| 36 | 3-Cl | H | 2 | $-NH-(CH_2)_3-NH-$ 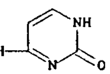 | base | 197-200 |
| 37 | $2-OCH_3$, 5-Cl | H | 2 | $-NH-(CH_2)_4-NH-$ 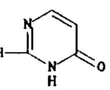 | base | 98-100 |
| 38 | $2-OCH_3$, 5-Cl | H | 3 | $-NH-CH_2-C(CH_3)_2-CH_2-NH-$ 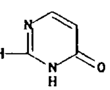 | base | 116-120 |
| 39 | $2-OCH_3$, 5-Cl | H | 2 | $-NH-CH_2-C(CH_3)_2-CH_2-NH-$ 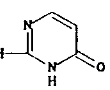 | base | 117-121 |

| N° | X | R | n | $-NR_1R_2$ | Sel ou base | F (°C) |
|---|---|---|---|---|---|---|
| 40 | $2-OCH_3$, $5-OCH_3$ | H | 2 | 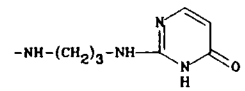 | base | 95-97 |
| 41 | $2-OCH_3$, $5-OCH_3$ | H | 3 | 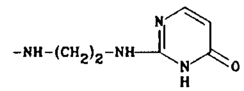 | base | 105-110 |
| 42 | $2-OCH_3$, $5-OCH_3$ | H | 2 | 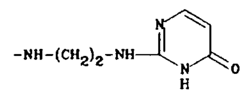 | base | 103-107 |
| 43 | $2-OCH_3$, 5-F | H | 2 | 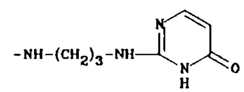 | base | 120-122 |
| 44 | $2-OCH_3$, 5-Cl | H | 2 | 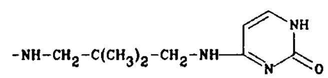 | base | 125-128 |
| 45 | $2-OCH_3$, 5-F | H | 2 | 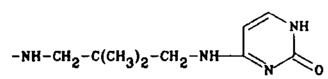 | base | 184-187 |
| 46 | $2-OCH_3$, 5-Cl | H | 3 | 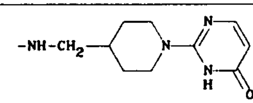 | $\frac{1}{2}C_2H_2O_4$, $\frac{1}{2}H_2O$ | 138-142 |
| 47 | $2-OCH_3$, 5-Cl | H | 3 | 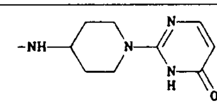 | $C_2H_2O_4$, $H_2O$ | 136-140 |
| 48 | $2-OCH_3$, 5-Cl | H | 2 | 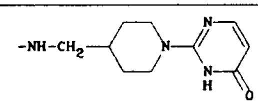 | $C_2H_2O_4$, $\frac{1}{2}H_2O$ | 155-160 |
| 49 | $2-OCH_3$, 5-Cl | H | 2 | 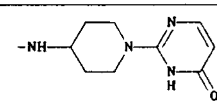 | base | 112-116 |

| N° | X | R | n | $-NR_1R_2$ | Sel ou base | F (°C) |
|---|---|---|---|---|---|---|
| 50 | 2-$OCH_3$, 5-Cl | H | 2 | 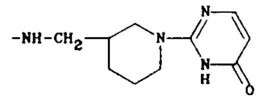 | base | 104-108 |
| 51 | 2-$OCH_3$, 5-Cl | H | 3 | 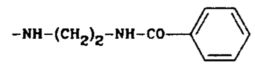 | base | 124-127 |
| 52 | 2-$OCH_3$, 5-Cl | H | 3 | 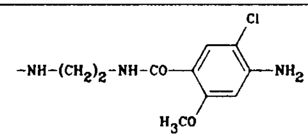 | $C_2H_2O_4$, ½$H_2O$ | 153-157 |
| 53 | 2-$OCH_3$, 5-Cl | H | 3 | 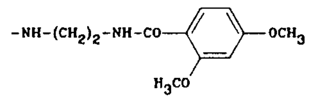 | $C_2H_2O_4$ | 113-117 |
| 54 | 2-$OCH_3$, 5-Cl | H | 3 | 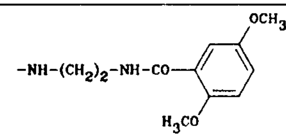 | $C_2H_2O_4$ | 106-110 |
| 55 | 2-$OCH_3$, 5-Cl | H | 3 | 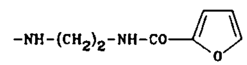 | $C_2H_2O_4$ | 126-130 |
| 56 | 2-$OCH_3$, 5-Cl | H | 3 | 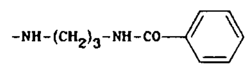 | ½$C_2H_2O_4$ | 172-175 |
| 57 | 2-$OCH_3$, 5-Cl | H | 3 | 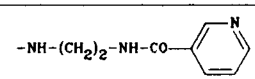 | $C_2H_2O_4$ | 118-122 |
| 58 | 2-$OCH_3$, 5-Cl | H | 3 | 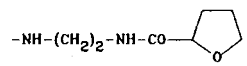 | $C_2H_2O_4$ | 151-155 |
| 59 | 2-$OCH_3$, 5-Cl | H | 2 | 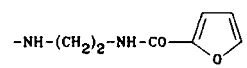 | $C_2H_2O_4$ | 128-132 |
| 60 | 3-Cl | H | 3 | $-N(CH_3)_2$ | 2HCl | 194-198 |
| 61 | 2-$OCH_3$, 5-Cl | H | 3 | $-N(CH_3)_2$ | HCl | 196-199 |

| N° | X | R | n | $-NR_1R_2$ | Sel ou base | F (°C) |
|---|---|---|---|---|---|---|
| 62 | 3-Cl | H | 3 | 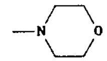 | 2HCl | 194-195 |
| 63 | 2-$OCH_3$, 5-Cl | H | 3 | 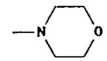 | 2HCl | 194-197 |
| 64 | 3-Cl | H | 3 | 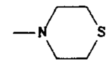 | 2HCl | 192-196 |
| 65 | 2-$OCH_3$, 5-Cl | H | 3 | 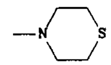 | 2HCl | 166-169 |
| 66 | 2-$OCH_3$, 5-Cl | H | 3 | 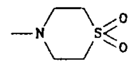 | base | 98,5-99,5 |
| 67 | 2-$OCH_3$, 5-Cl | H | 3 | 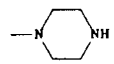 | 2HCl | 189-191 |
| 68 | 3-Cl | H | 3 | 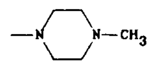 | 2HCl, $H_2O$ | 162-165 |
| 69 | 2-$OCH_3$, 5-Cl | H | 3 | 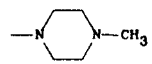 | 2HCl, ½$H_2O$ | 253-256 |
| 70 | 3-Cl | H | 3 | 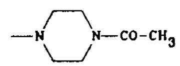 | HCl | 179-181 |
| 71 | 3-Cl | H | 2 | 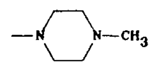 | 2HCl | 254-257 |
| 72 | 2-$OCH_3$, 5-Cl | H | 2 | 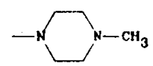 | 2HCl | 255-257 |
| 73 | 2-$OCH_3$, 5-Cl | H | 3 | 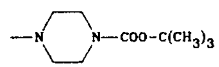 | HCl | 206,5-207,5 |
| 74 | 3-Cl | H | 3 | 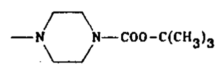 | HCl | 207-209 |

| N° | X | R | n | $-NR_1R_2$ | Sel ou base | F (°C) |
|---|---|---|---|---|---|---|
| 75 | $2-OCH_3$, 5-Cl | H | 2 | 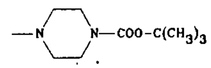 | HCl | 210-211,5 |
| 76 | $2-OCH_3$, 5-Cl | H | 3 | 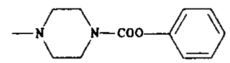 | HCl | 162-165 |
| 77 | $2-OCH_3$, 5-Cl | H | 3 | 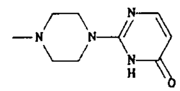 | base | 107-110 |
| 78 | $2-OCH_3$, 5-Cl | H | 2 | 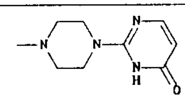 | base | 110-113 |
| 79 | $2-OCH_3$, 5-Cl | H | 2 | 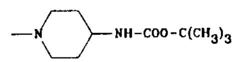 | HCl | 194-197 |
| 80 | $2-OCH_3$, 5-Cl | H | 2 | 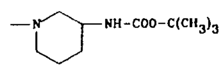 | base | 72-76 |
| 81 | $2-OCH_3$, 5-Cl | H | 2 | 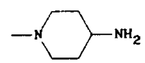 | base | solide amorphe |
| 82 | $2-OCH_3$, 5-Cl | H | 2 | 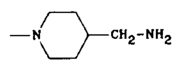 | base | solide amorphe |
| 83 | $2-OCH_3$, 5-Cl | H | 2 | 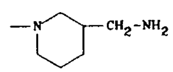 | base | solide amorphe |
| 84 | $2-OCH_3$, 5-Cl | H | 3 | 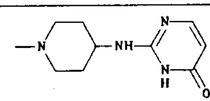 | base | 128-131 |
| 85 | $2-OCH_3$, 5-Cl | H | 2 | 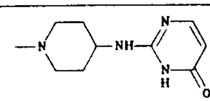 | base | 114-117 |
| 86 | $2-OCH_3$, 5-Cl | H | 3 | 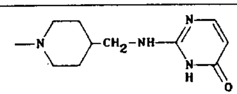 | $C_2H_2O_4$, $H_2O$ | 150-154 |

| N° | X | R | n | $-NR_1R_2$ | Sel ou base | F (°C) |
|---|---|---|---|---|---|---|
| 87 | $2-OCH_3$, 5-Cl | H | 2 | 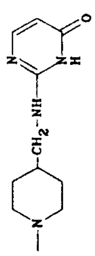 | $C_2H_2O_4$ | 147-150 |
| 88 | $2-OCH_3$, 5-Cl | H | 2 | 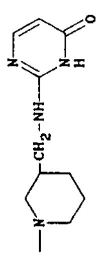 | base | 105-108 |
| 89 | $2-OCH_3$, 5-Cl | H | 3 | 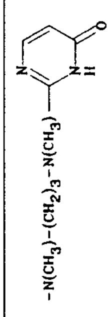 | base | 87-92 |
| 90 | 3-Cl | $CH_3$ | 3 | $-N(CH_3)_2$ | HCl | 163-166 |

<u>Légende</u>

Dans la colonne "Sel ou base", HCl désigne un chlorhydrate, 2HCl désigne un dichlorhydrate, $C_2H_2O_4$ désigne un oxalate acide, $\frac{1}{2}C_2H_2O_4$ désigne un oxalate neutre ; $H_2O$ désigne un composé hydraté, et $\frac{1}{2}H_2O$ désigne un composé hémihydraté.

Les composés de l'invention ont fait l'objet d'études quant à leur activité antagoniste des récepteurs $\alpha_1$-adrénergiques au niveau du bas appareil urinaire.

Leur activité *in vitro* a été étudiée sur l'urètre isolé de lapin.

On prépare des anneaux d'urètre de lapin adulte selon la méthode de Ueda et al., *Eur. J. Pharmacol.*, (1984), **103**, 249-254, puis, après sensibilisation à la noradrénaline, on détermine la courbe concentration-

réponse à la phényléphrine, en absence et en présence de composé à étudier.
On évalue la puissance de l'antagonisme $\alpha_1$-adrénergique de chaque composé par calcul du $pA_2$, antilogarithme de la concentration molaire d'antagoniste en présence de laquelle la concentration d'agoniste doit être doublée pour engendrer le même effet qu'en son absence.
Les $pA_2$ des composés sont de l'ordre de 5,5 à 9.

L'activité *in vivo* des composés de l'invention a été étudiée quant à leur effet sur l'hypertonie urétrale engendrée par la stimulation des fibres sympathiques du nerf hypogastrique chez le chat anesthésié.
On anesthésie des chats mâles adultes au pentobarbital sodique, et on les prépare selon la méthode de Theobald, *J. Auton. Pharmac.,* (1983), **3**, 235-239, afin d'obtenir une hypertonie urétrale par stimulation des fibres sympathiques du nerf hypogastrique. On note les réponses contractiles de l'urètre à la stimulation électrique du nerf hypogastrique avant et après administration intraveineuse des composés à étudier, à doses cumulatives de 1 à 1000 µg/kg.
On évalue la puissance de l'antagonisme $\alpha_1$-adrénergique de chaque composé par calcul de la $DI_{50}$, dose qui inhibe de 50% l'hypertonie urétrale.
Les $DI_{50}$ des composés de l'invention sont de l'ordre de 0,01 à 1 mg/kg.

Les résultats des essais montrent que les composés de l'invention montrent, *in vitro*, une activité antagoniste des récepteurs $\alpha_1$-adrénergiques des muscles lisses du bas appareil urinaire (urètre) stimulés par un agoniste $\alpha_1$-adrénergique (phényléphrine). *In vivo*, ils inhibent l'hypertonie urétrale engendrée par la stimulation nerveuse sympathique.

Les composés de l'invention peuvent donc être utilisés pour le traitement symptomatique des maladies et affections impliquant une hyperactivité du système $\alpha$-adrénergique au niveau du bas appareil urinaire, et notamment pour le traitement de l'hypertrophie bénigne de la prostate, de la dysurie, de la pollakiurie.

A cet effet ils peuvent être présentés sous toutes formes appropriées à l'administration entérale ou parentérale, associés à des excipients pharmaceutiques, par exemple sous forme de comprimés, dragées, gélules, capsules, solutions ou suspensions buvables ou injectables, suppositoires, étant dosés pour permettre une dose journalière de 0,5 à 500 mg de substance active.

**Revendications**

**Revendications pour les Etats contractants suivants : AT, BE, CH, DE, DK, FR, GB, IT, LI, LU, MC, NL, PT, SE**

**1.** Composé répondant à la formule générale (I)

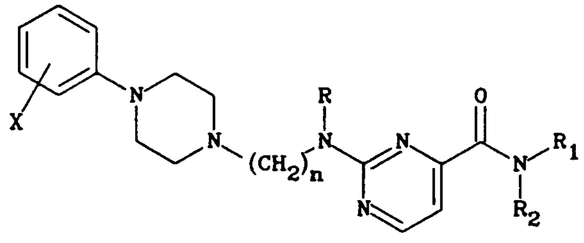

(I)

dans laquelle

- n représente le nombre 2 ou 3,
- X représente un ou plusieurs atomes ou groupes choisis parmi les suivants : hydrogène, fluor, chlore, méthoxy,
- R représente un atome d'hydrogène ou un groupe méthyle,
- $R_1$ représente un atome d'hydrogène ou un groupe méthyle,
- $R_2$ représente
  - un groupe alkyle en $C_1$-$C_6$,
  - un groupe hydroxyalkyle en $C_2$-$C_3$,
  - un groupe (hydroxy)(méthoxy)alkyle en $C_2$-$C_3$,
  - un groupe diméthoxyalkyle en $C_2$-$C_3$,
  - un groupe 2-(aminosulfonyl)éthyle,
  - un groupe 2-(méthylsulfonyl)éthyle,
  - un groupe 2-(méthylsulfonylamino)éthyle,

- un groupe de formule générale $-CH_2-CO-NY_1Y_2$ (dans laquelle $Y_1$ et $Y_2$, indépendamment l'un de l'autre, représentent chacun un atome d'hydrogène ou un groupe alkyle en $C_1$-$C_6$),
- un groupe de formule générale $-(CH_2)_2-Ar$ (dans laquelle Ar représente un groupe phényle éventuellement substitué par un ou plusieurs groupes méthoxy ou aminosulfonyle),
- un groupe de formule générale

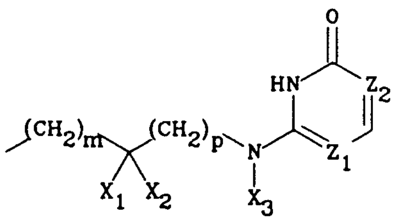

ou (tautomère)

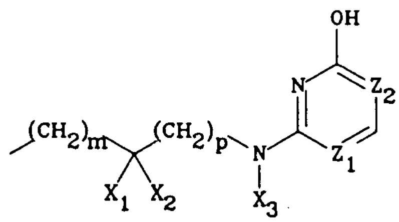

dans lesquelles m représente le nombre 0 ou 1, p représente le nombre 1 ou 2, l'un des symboles $Z_1$ et $Z_2$ représente un groupe CH et l'autre représente un atome d'azote et, soit $X_1$, $X_2$ et $X_3$ représentent chacun un atome d'hydrogène, soit $X_1$ et $X_2$ représentent chacun un groupe méthyle et $X_3$ représente un atome d'hydrogène, soit $X_1$ représente un atome d'hydrogène et $X_2$ et $X_3$ forment ensemble une chaîne de formule générale $-(CH_2)_{4-p}$ (p étant tel que défini ci-dessus), ou
- un groupe de formule générale

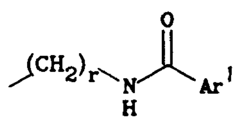

(dans laquelle r représente le nombre 2 ou 3 et Ar' représente soit un groupe phényle éventuellement substitué par un ou plusieurs groupes chloro, méthoxy ou amino, soit un groupe furan-2-yle, soit un groupe tétrahydrofuran-2-yle, soit un groupe pyridin-3-yle),

ou bien encore $R_1$ et $R_2$ forment, avec l'atome d'azote qui les porte,
- un groupe de formule générale

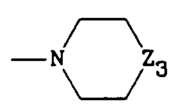

dans laquelle $Z_3$ représente un atome d'oxygène ou de soufre, un groupe sulfonyle ou un groupe de formule générale $N-R_4$ dans laquelle $R_4$ représente un atome d'hydrogène, un groupe méthyle, un groupe acétyle, un groupe tert.-butyloxycarbonyle, un groupe phényloxycarbonyle ou un groupe de formule générale

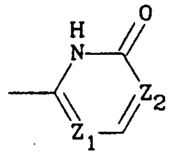

ou (tautomère)

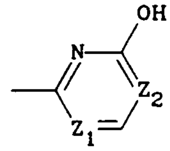

dans laquelle $Z_1$ et $Z_2$ sont tels que définis ci-dessus, ou

- un groupe de formule générale

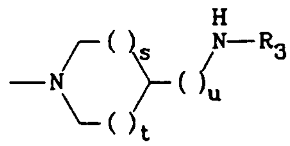

dans laquelle s représente soit le nombre 0, auquel cas t représente le nombre 2, soit le nombre 1, auquel cas t représente le nombre 1, u représente le nombre 0 ou 1 et $R_3$ représente un atome d'hydrogène, un groupe tert.-butyloxycarbonyle, un groupe phényloxycarbonyle ou un groupe de formule générale

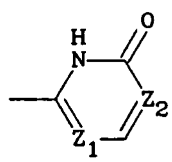 ou (tautomère) 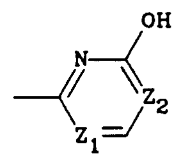

dans laquelle $Z_1$ et $Z_2$ sont tels que définis ci-dessus,

à l'état de base libre ou de sel d'addition à un acide pharmaceutiquement acceptable.

**2.** Composé selon la revendication 1, caractérisé en ce que $R_2$ représente un groupe de formule générale

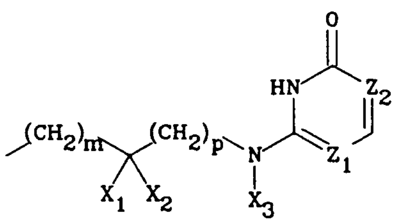 ou (tautomère) 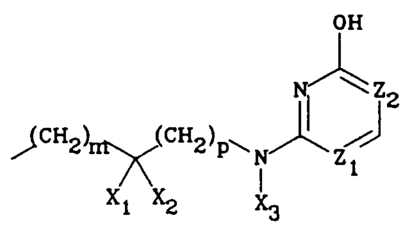

dans lesquelles m, p, $Z_1$, $Z_2$, $X_1$, $X_2$ et $X_3$ sont tels que définis dans la revendication 1.

**3.** Composé selon la revendication 1, caractérisé en ce que $R_1$ et $R_2$ forment, avec l'atome d'azote qui les porte, un groupe de formule générale

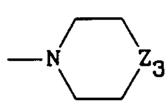

dans laquelle $Z_3$ représente un groupe de formule générale N-$R_4$ dans laquelle $R_4$ représente un groupe de formule générale

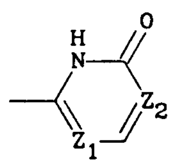 ou (tautomère) 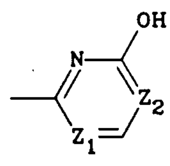

dans laquelle $Z_1$ et $Z_2$ sont tels que définis dans la revendication 1.

**4.** Composé selon la revendication 1, caractérisé en ce que $R_1$ et $R_2$ forment, avec l'atome d'azote qui les porte, un groupe de formule générale

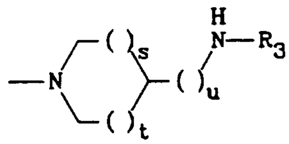

dans laquelle s représente soit le nombre 0, auquel cas t représente le nombre 2, soit le nombre 1, auquel cas t représente le nombre 1, u représente le nombre 0 ou 1 et $R_3$ représente un groupe de formule générale

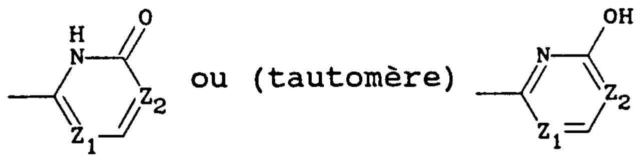

dans laquelle $Z_1$ et $Z_2$ sont tels que définis dans la revendication 1.

5. Composé selon la revendication 1, caractérisé en ce que $R_1$ et $R_2$ forment, avec l'atome d'azote qui les porte, un groupe de formule générale

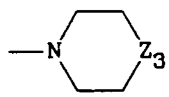

dans laquelle $Z_3$ représente un groupe de formule générale N-$R_4$ dans laquelle $R_4$ représente un atome d'hydrogène, un groupe méthyle, un groupe acétyle, un groupe tert.-butyloxycarbonyle ou un groupe phényloxycarbonyle.

6. Composé selon la revendication 1, caractérisé en ce que $R_2$ représente un groupe de formule générale

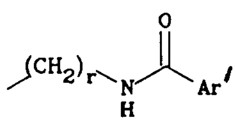

dans laquelle r représente le nombre 2 ou 3 et Ar' représente soit un groupe phényle éventuellement substitué par un ou plusieurs groupes chloro, méthoxy ou amino, soit un groupe furan-2-yle, soit un groupe tétrahydrofuran-2-yle, soit un groupe pyridin-3-yle.

7. Composé selon la revendication 1, caractérisé en ce que $R_2$ représente un groupe (hydroxy)(méthoxy)-alkyle en $C_2$-$C_3$ ou un groupe diméthoxyalkyle en $C_2$-$C_3$.

8. Procédé de préparation d'un composé selon la revendication 1, caractérisé en ce qu'on transforme un amide de formule générale (II)

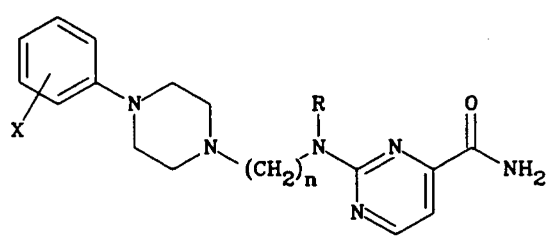

(II)

dans laquelle n, X et R sont tels que définis dans la revendication 1, en ester de formule générale (III)

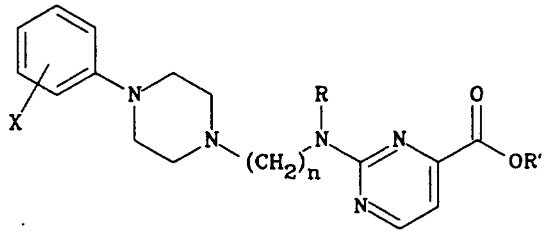

(III)

dans laquelle R′ représente un groupe alkyle en $C_1$-$C_4$, par réaction avec un alcool aliphatique, en présence d'un acide, puis on fait réagir l'ester ainsi obtenu avec une amine de formule générale (IV)

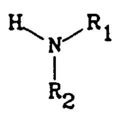

(IV)

dans laquelle $R_1$ et $R_2$ sont tels que définis dans la revendication 1, directement lorsque ladite amine est primaire ($R_1$ = H), ou en préparant préalablement l'amidure de diméthylaluminium correspondant au moyen de triméthylaluminium lorsque ladite amine est secondaire ($R_1$ = $CH_3$).

**9.** Médicament caractérisé en ce qu'il consiste en un composé selon la revendication 1.

**10.** Composition pharmaceutique caractérisée en ce qu'elle contient un composé selon la revendication 1, associé à un excipient pharmaceutique.

**Revendication pour les Etats contractants suivants : ES, GR**

**1.** Procédé de préparation d'un composé répondant à la formule générale (I)

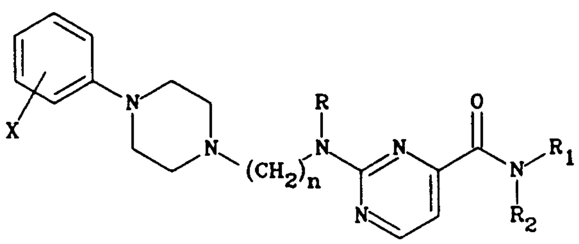

(I)

dans laquelle

- n représente le nombre 2 ou 3,
- X représente un ou plusieurs atomes ou groupes choisis parmi les suivants : hydrogène, fluor, chlore, méthoxy,
- R représente un atome d'hydrogène ou un groupe méthyle,
- $R_1$ représente un atome d'hydrogène ou un groupe méthyle,
- $R_2$ représente
  - un groupe alkyle en $C_1$-$C_6$,
  - un groupe hydroxyalkyle en $C_2$-$C_3$,
  - un groupe (hydroxy)(méthoxy)alkyle en $C_2$-$C_3$,
  - un groupe diméthoxyalkyle en $C_2$-$C_3$,
  - un groupe 2-(aminosulfonyl)éthyle,
  - un groupe 2-(méthylsulfonyl)éthyle,
  - un groupe 2-(méthylsulfonylamino)éthyle,

- un groupe de formule générale $-CH_2-CO-NY_1Y_2$ (dans laquelle $Y_1$ et $Y_2$, indépendamment l'un de l'autre, représentent chacun un atome d'hydrogène ou un groupe alkyle en $C_1$-$C_6$),
- un groupe de formule générale $-(CH_2)_2$-Ar (dans laquelle Ar représente un groupe phényle éventuellement substitué par un ou plusieurs groupes méthoxy ou aminosulfonyle),
- un groupe de formule générale

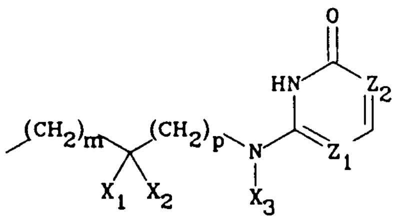

ou (tautomère)

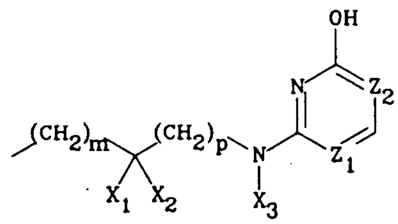

dans lesquelles m représente le nombre 0 ou 1, p représente le nombre 1 ou 2, l'un des symboles $Z_1$ et $Z_2$ représente un groupe CH et l'autre représente un atome d'azote et, soit $X_1$, $X_2$ et $X_3$ représentent chacun un atome d'hydrogène, soit $X_1$ et $X_2$ représentent chacun un groupe méthyle et $X_3$ représente un atome d'hydrogène, soit $X_1$ représente un atome d'hydrogène et $X_2$ et $X_3$ forment ensemble une chaîne de formule générale $-(CH_2)_{4-p}$ (p étant tel que défini ci-dessus), ou

- un groupe de formule générale

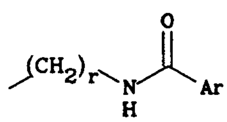

(dans laquelle r représente le nombre 2 ou 3 et Ar' représente soit un groupe phényle éventuellement substitué par un ou plusieurs groupes chloro, méthoxy ou amino, soit un groupe furan-2-yle, soit un groupe tétrahydrofuran-2-yle, soit un groupe pyridin-3-yle),

ou bien encore $R_1$ et $R_2$ forment, avec l'atome d'azote qui les porte,

- un groupe de formule générale

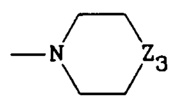

dans laquelle $Z_3$ représente un atome d'oxygène ou de soufre, un groupe sulfonyle ou un groupe de formule générale N-$R_4$ dans laquelle $R_4$ représente un atome d'hydrogène, un groupe méthyle, un groupe acétyle, un groupe tert.-butyloxycarbonyle, un groupe phényloxycarbonyle ou un groupe de formule générale

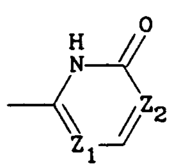

ou (tautomère)

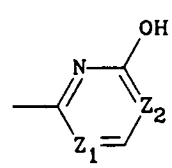

dans laquelle $Z_1$ et $Z_2$ sont tels que définis ci-dessus, ou

- un groupe de formule générale

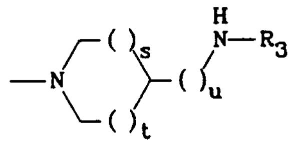

dans laquelle s représente soit le nombre 0, auquel cas t représente le nombre 2, soit le nombre 1, auquel cas t représente le nombre 1, u représente le nombre 0 ou 1 et $R_3$ représente un atome d'hydrogène, un groupe tert.-butyloxycarbonyle, un groupe phényloxycarbonyle ou un groupe de formule générale

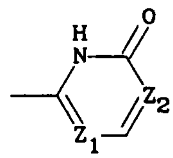
ou (tautomère)
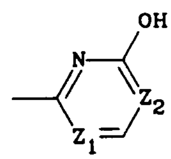

dans laquelle $Z_1$ et $Z_2$ sont tels que définis ci-dessus,

Procédé caractérisé en ce qu'on transforme un amide de formule générale (II)

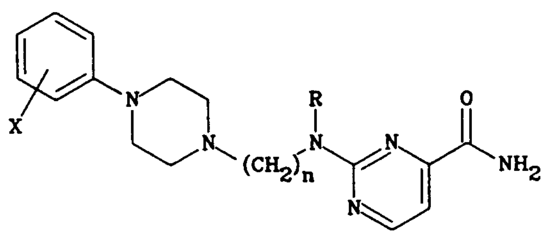
(II)

dans laquelle n, X et R sont tels que définis ci-dessus, en ester de formule générale (III)

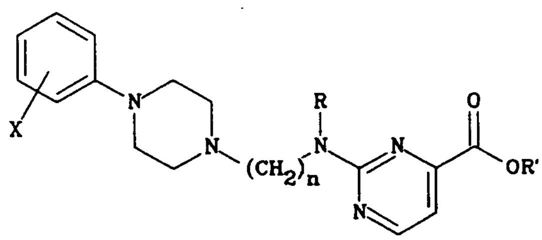
(III)

dans laquelle R′ représente un groupe alkyle en $C_1$-$C_4$, par réaction avec un alcool aliphatique, en présence d'un acide, puis on fait réagir l'ester ainsi obtenu avec une amine de formule générale (IV)

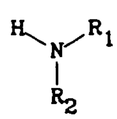
(IV)

dans laquelle $R_1$ et $R_2$ sont tels que définis ci-dessus, directement lorsque ladite amine est primaire ($R_1$ = H), ou en préparant préalablement l'amidure de diméthylaluminium correspondant au moyen de triméthylaluminium lorsque ladite amine est secondaire ($R_1$ = $CH_3$).

## Claims

**Claims for the following Contracting States : AT, BE, CH, DE, DK, FR, GB, IT, LI, LU, MC, NL, PT, SE**

**1.** Compound corresponding to the general formula (I)

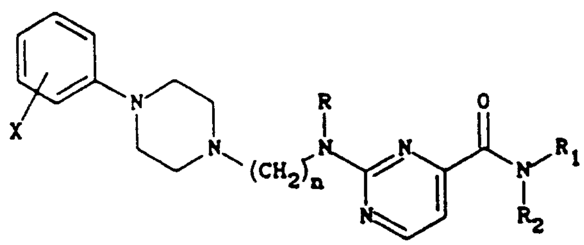

(I)

in which

- n represents the number 2 or 3,
- X represents one or more atoms or groups chosen from the following: hydrogen, fluorine, chlorine, methoxy,
- R represents a hydrogen atom or a methyl group,
- $R_1$ represents a hydrogen atom or a methyl group,
- $R_2$ represents
  - a $C_1$-$C_6$ alkyl group,
  - a $C_2$-$C_3$ hydroxyalkyl group,
  - a (hydroxy) (methoxy) $C_2$-$C_3$ alkyl group,
  - a dimethoxy $C_2$-$C_3$ alkyl group,
  - a 2-(aminosulphonyl)ethyl group,
  - a 2-(methylsulphonyl)ethyl group,
  - a 2-(methylsulphonylamino)ethyl group,
  - a group of general formula $-CH_2-CO-NY_1Y_2$ (in which $Y_1$ and $Y_2$, independently of each other, each represent a hydrogen atom or a $C_1$-$C_6$ alkyl group),
  - a group of general formula $-(CH_2)_2-Ar$ (in which Ar represents a phenyl group, optionally substituted by one or more methoxy or aminosulphonyl groups),
  - a group of general formula

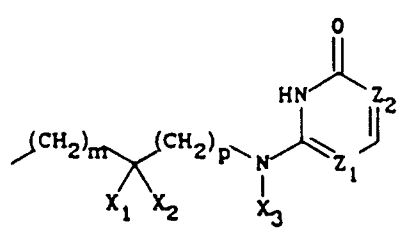

or (tautomer)

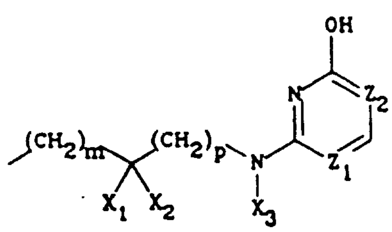

in which m represents the number 0 or 1, p represents the number 1 or 2, one of the symbols $Z_1$ and $Z_2$ represents a CH group and the other represents a nitrogen atom and either $X_1$, $X_2$ and $X_3$ each represent a hydrogen atom or $X_1$ and $X_2$ each represent

a methyl group and $X_3$ represents a hydrogen atom or $X_1$ represents a hydrogen atom and $X_2$ and $X_3$ together form a chain of general formula $-(CH_2)_{4-p}$ (p being as defined above), or

- a group of general formula

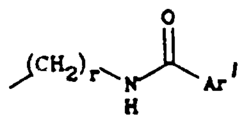

(in which r represents the number 2 or 3 and Ar' represents either a phenyl group, optionally substituted by one or more chloro, methoxy or amino groups, or a 2-furanyl group or a 2-tetrahydrofuranyl group or a 3-pyridinyl group),

or else $R_1$ and $R_2$ form, with the nitrogen atom which carries them,

- a group of general formula

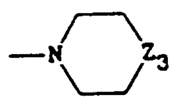

in which $Z_3$ represents an oxygen or sulphur atom, a sulphonyl group or a group of general formula $N\text{-}R_4$ in which $R_4$ represents a hydrogen atom, a methyl group, an acetyl group, a tert-butyloxycarbonyl group, a phenyloxycarbonyl group or a group of general formula

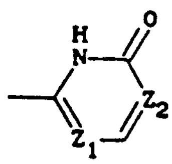 or (tautomer) 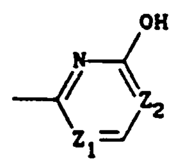

in which $Z_1$ and $Z_2$ are as defined above, or

- a group of general formula

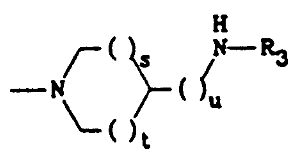

in which s represents either the number 0, in which case t represents the number 2, or the number 1, in which case t represents the number 1, u represents the number 0 or 1 and $R_3$ represents a hydrogen atom, a tert-butyloxycarbonyl group, a benzyloxycarbonyl group or a group of general formula

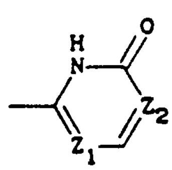 or (tautomer) 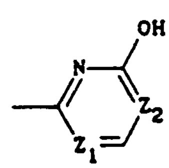

in which $Z_1$ and $Z_2$ are as defined above,
in the form of a free base or of an addition salt with a pharmaceutically acceptable acid.

**2.** Compound according to Claim 1, characterised in that $R_2$ represents a group of general formula

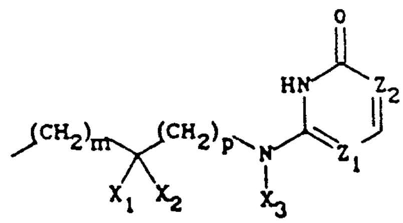

or (tautomer)

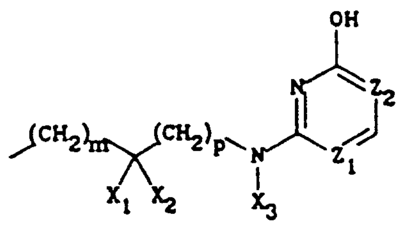

in which m, p, $Z_1$, $Z_2$, $X_1$, $X_2$ and $X_3$ are as defined in Claim 1.

**3.** Compound according to Claim 1, characterised in that $R_1$ and $R_2$ form, with the nitrogen atom which carries them, a group of general formula

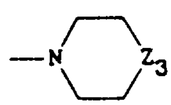

in which $Z_3$ represents a group of general formula N-$R_4$ in which $R_4$ represents a group of general formula

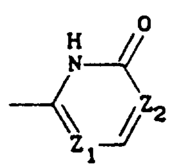

or (tautomer)

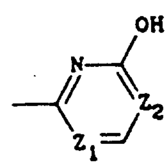

in which $Z_1$ and $Z_2$ are as defined in Claim 1.

**4.** Compound according to Claim 1, characterised in that $R_1$ and $R_2$ form, with the nitrogen atom which carries them, a group of general formula

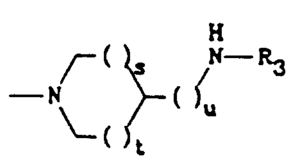

in which s represents either the number 0, in which case t represents the number 2, or the number 1, in which case t represents the number 1, u represents the number 0 or 1 and $R_3$ represents a group of general formula

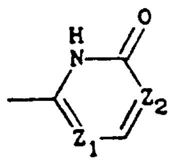 or (tautomer) 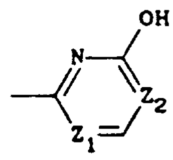

in which $Z_1$ and $Z_2$ are as defined in Claim 1.

**5.** Compound according to Claim 1, characterised in that $R_1$ and $R_2$ form, with the nitrogen atom which carries them, a group of general formula

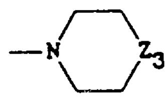

in which $Z_3$ represents a group of general formula $N-R_4$ in which $R_4$ represents a hydrogen atom, a methyl group, an acetyl group, a tert-butyloxycarbonyl group or a phenyloxycarbonyl group.

**6.** Compound according to Claim 1, characterised in that $R_2$ represents a group of general formula

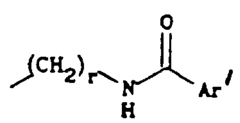

in which r represents the number 2 or 3 and Ar' represents either a phenyl group, optionally substituted by one or more chloro, methoxy or amino groups, a 2-furanyl group, a 2-tetrahydrofuranyl group or a 3-pyridinyl group.

**7.** Compound according to Claim 1, characterised in that $R_2$ represents a (hydroxy)(methoxy) $C_2$-$C_3$ alkyl group or a dimethoxy $C_2$-$C_3$ alkyl group.

**8.** Process for preparing a compound according to Claim 1, characterised in that an amide of general formula (II)

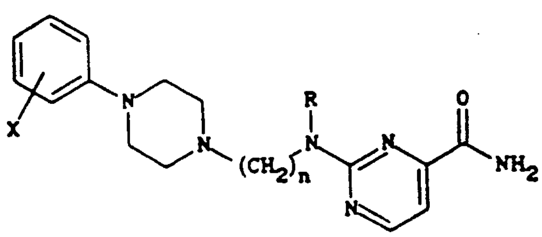 (II)

in which n, X and R are as defined in Claim 1, is converted to an ester of general formula (III)

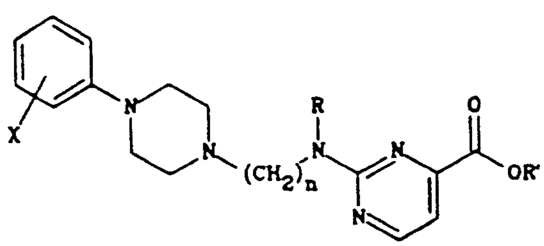 (III)

in which R' represents a $C_1$-$C_4$ alkyl group, by reaction with an aliphatic alcohol in the presence of

an acid and the ester thus obtained is then reacted with an amine of general formula (IV)

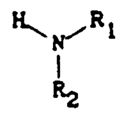

(IV)

in which $R_1$ and $R_2$ are as defined in Claim 1, directly when the said amine is primary ($R_1$ = H) or by preparing the corresponding dimethylaluminium amide beforehand by means of trimethylaluminium when the said amine is secondary ($R_1 = CH_3$).

**9.** Medicament characterised in that it consists of a compound according to Claim 1.

**10.** Pharmaceutical composition characterised in that it contains a compound according to Claim 1 in combination with a pharmaceutical excipient.

**Claim for the following Contracting States : ES, GR**

**1.** Process for the preparation of a compound corresponding to the general formula (I)

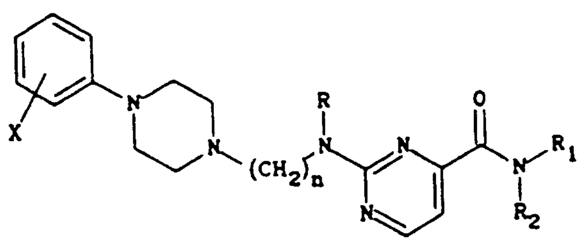

(I)

in which

- n represents the number 2 or 3,
- X represents one or more atoms or groups chosen from the following: hydrogen, fluorine, chlorine, methoxy,
- R represents a hydrogen atom or a methyl group,
- $R_1$ represents a hydrogen atom or a methyl group,
- $R_2$ represents
  - a $C_1$-$C_6$ alkyl group,
  - a $C_2$-$C_3$ hydroxyalkyl group,
  - a (hydroxy)(methoxy) $C_2$-$C_3$ alkyl group,
  - a dimethoxy $C_2$-$C_3$ alkyl group,
  - a 2-(aminosulphonyl)ethyl group,
  - a 2-(methylsulphonyl)ethyl group,
  - a 2-(methylsulphonylamino)ethyl group,
  - a group of general formula $-CH_2-CO-NY_1Y_2$ (in which $Y_1$ and $Y_2$, independently of each other, each represent a hydrogen atom or a $C_1$-$C_6$ alkyl group),
  - a group of general formula $-(CH_2)_2-Ar$ (in which Ar represents a phenyl group, optionally substituted by one or more methoxy or aminosulphonyl groups),

- a group of general formula

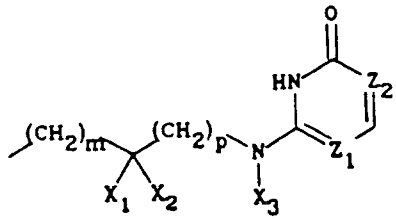

or (tautomer)

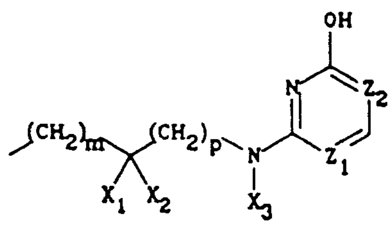

in which m represents the number 0 or 1, p represents the number 1 or 2, one of the symbols $Z_1$ and $Z_2$ represents a CH group and the other represents a nitrogen atom and either $X_1$, $X_2$ and $X_3$ each represent a hydrogen atom or $X_1$ and $X_2$ each represent a methyl group and $X_3$ represents a hydrogen atom or $X_1$ represents a hydrogen atom and $X_2$ and $X_3$ together form a chain of general formula $-(CH_2)_{4-p}$ (p being as defined above), or

- a group of general formula

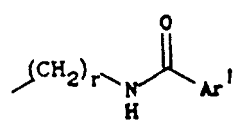

(in which r represents the number 2 or 3 and Ar' represents either a phenyl group, optionally substituted by one or more chloro, methoxy or amino groups, or a 2-furanyl group or a 2-tetrahydrofuranyl group or a 3-pyridinyl group),

or else $R_1$ and $R_2$ form, with the nitrogen atom which carries them,

- a group of general formula

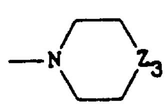

in which $Z_3$ represents an oxygen or sulphur atom, a sulphonyl group or a group of general formula $N-R_4$ in which $R_4$ represents a hydrogen atom, a methyl group, an acetyl group, a tert-butyloxycarbonyl group, a phenyloxycarbonyl group or a group of general formula

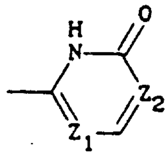 or (tautomer) 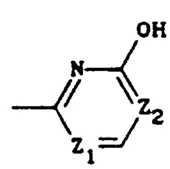

in which $Z_1$ and $Z_2$ are as defined above, or

- a group of general formula

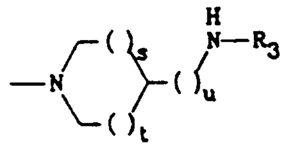

in which s represents either the number 0, in which case t represents the number 2, or the number 1, in which case t represents the number 1, u represents the number 0 or 1 and $R_3$ represents a hydrogen atom, a tert-butyloxycarbonyl group, a benzyloxycarbonyl group or a group of general formula

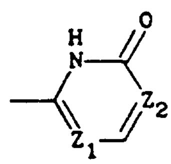 or (tautomer) 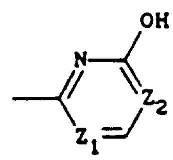

in which $Z_1$ and $Z_2$ are as defined above,
which process is characterised in that an amide of general formula (II)

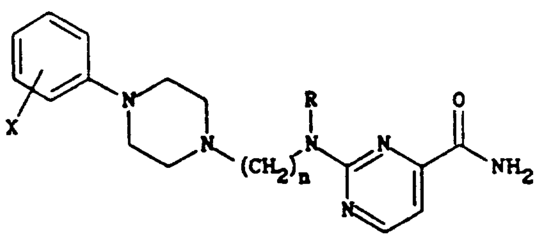

(II)

in which n, X and R are as defined above, is converted to an ester of general formula (III)

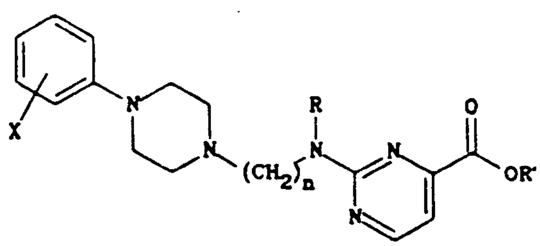

(III)

in which R' represents a $C_1$-$C_4$ alkyl group, by reaction with an aliphatic alcohol in the presence of an acid and the ester thus obtained is then reacted with an amine of general formula (IV)

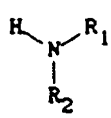

(IV)

in which $R_1$ and $R_2$ are as defined above, directly when the said amine is primary ($R_1$ = H) or by preparing the corresponding dimethylaluminium amide beforehand by means of trimethylaluminium when the said amine is secondary ($R_1 = CH_3$).

**Patentansprüche**

**Patentansprüche für folgende Vertragsstaaten : AT, BE, CH, DE, DK, FR, GB, IT, LI, LU, MC, NL, PT, SE**

**1.** Verbindung der allgemeinen Formel (I)

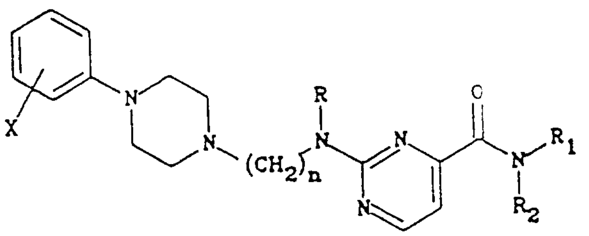

(I)

in der

- n die Zahl 2 oder 3,
- X eines oder mehrere Atome oder Gruppen ausgewählt aus: Wasserstoffatomen, Fluoratomen, Chloratomen und Methoxygruppen,
- R ein Wasserstoffatom oder eine Methylgruppe,
- $R_1$ ein Wasserstoffatom oder eine Methylgruppe,
- $R_2$
  - eine $C_1$-$C_6$-Alkylgruppe,
  - eine $C_2$-$C_3$-Hydroxyalkylgruppe,
  - eine $C_2$-$C_3$-(Hydroxy)-(methoxy)-alkylgruppe,
  - eine $C_2$-$C_3$-Dimethoxyalkylgruppe,
  - eine 2-(Aminosulfonyl)-ethylgruppe,
  - eine 2-(Methylsulfonyl)-ethylgruppe,
  - eine 2-(Methylsulfonylamino)-ethylgruppe,
  - eine Gruppe der allgemeinen Formel $-CH_2-CO-NY_1Y_2$ (worin $Y_1$ und $Y_2$ unabhängig voneinander jeweils ein Wasserstoffatom oder eine $C_1$-$C_6$-Alkylgruppe darstellen),
  - eine Gruppe der allgemeinen Formel $-(CH_2)_2-Ar$ (worin Ar eine Phenylgruppe, die gegebenenfalls durch eine oder mehrere Methoxy- oder Aminosulfonylgruppen substituiert ist, darstellt),
  - eine Gruppe der allgemeinen Formel

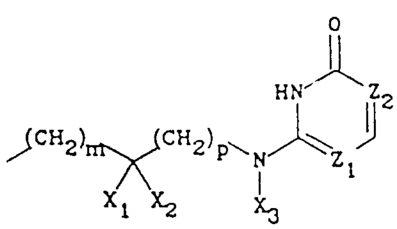

oder (Tautomer)

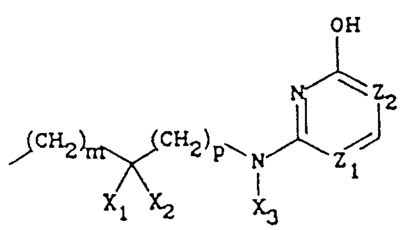

worin m die Zahl 0 oder 1, p die Zahl 1 der 2, eines der Symbole $Z_1$ und $Z_2$ eine CH-Gruppe und das andere ein Stickstoffatom oder $X_1$, $X_2$ und $X_3$ jeweils ein Wasserstoffatom, oder $X_1$ und $X_2$ jeweils eine Methylgruppe und $X_3$ ein Wasserstoffatom, oder $X_1$ ein Wasserstoffatom und $X_2$ und $X_3$ gemeinsam eine Kette der allgemeinen Formel $-(CH_2)_{4-p}$ (worin p die oben angegebenen Bedeutungen besitzt) darstellen, oder

  - eine Gruppe der allgemeinen Formel

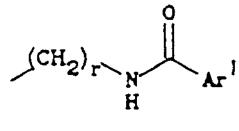

(in der r die Zahl 2 oder 3 und Ar' entweder eine Phenylgruppe, die gegebenenfalls durch eines oder mehrere Chloratome, Methoxygruppen oder Aminogruppen substituiert ist, oder eine Furan-2-yl-gruppe oder eine Tetrahydrofuran-2-yl-gruppe oder eine Pyridin-3-yl-gruppe darstellt),

oder schließlich $R_1$ und $R_2$ gemeinsam mit dem Stickstoffatom, welches sie trägt,

- eine Gruppe der allgemeinen Formel

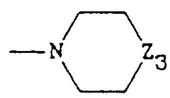

in der $Z_3$ ein Sauerstoffatom oder ein Schwefelatom, eine Sulfonylgruppe oder eine Gruppe der allgemeinen Formel N-$R_4$, worin $R_4$ ein Wasserstoffatom, eine Methylgruppe, eine Acetylgruppe, eine tert.-Butyloxycarbonylgruppe, eine Phenyloxycarbonylgruppe oder eine Gruppe der allgemeinen Formel

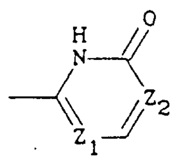

oder (Tautomer)

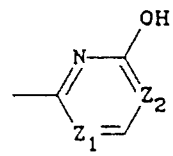

in der $Z_1$ und $Z_2$ die oben angegebenen Bedeutungen besitzen, darstellen, oder

- eine Gruppe der allgemeinen Formel

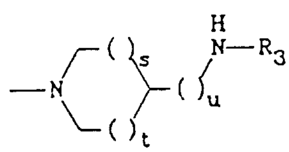

in der s entweder die Zahl 0, und in diesem Fall t die Zahl 2, oder die Zahl 1, und in diesem Fall t die Zahl 1, u die Zahl 0 oder 1 und $R_3$ ein Wasserstoffatom, eine tert.-Butyloxycarbonylgruppe, eine Phenyloxycarbonylgruppe oder eine Gruppe der allgemeinen Formel

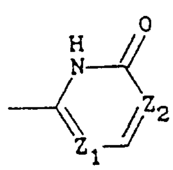

oder (Tautomer)

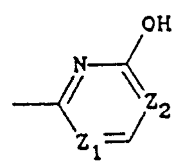

in der $Z_1$ und $Z_2$ die oben angegebenen Bedeutungen besitzen, bedeuten,

in Form der freien Base oder des Additionssalzes mit einer pharmazeutisch annehmbaren Säure.

2. Verbindung nach Anspruch 1, **dadurch gekennzeichnet**, daß $R_2$ eine Gruppe der allgemeinen Formel

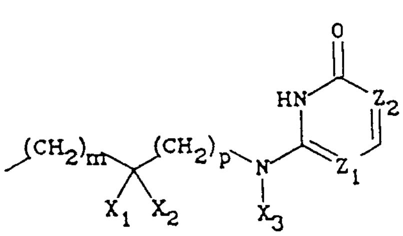

oder (Tautomer)

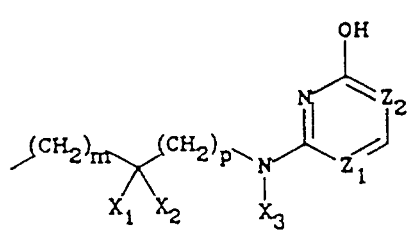

in der m, p, $Z_1$ $Z_2$, $X_1$, $X_2$ und $X_3$ die in Anspruch 1 angegebenen Bedeutungen besitzen, bedeutet

3. Verbindung nach Anspruch 1, **dadurch gekennzeichnet**, daß $R_1$ und $R_2$ zusammen mit dem Stickstoffatom, das sie trägt, eine Gruppe der allgemeinen Formel

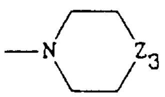

bilden, worin $Z_3$ eine Gruppe der allgemeinen Formel N-$R_4$ darstellt, worin $R_4$ eine Gruppe der allgemeinen Formel

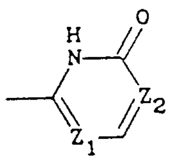

oder (Tautomer)

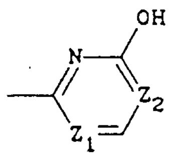

darstellt, worin $Z_1$ und $Z_2$ die in Anspruch 1 angegebenen Bedeutungen besitzen.

4. Verbindung nach Anspruch 1**, dadurch gekennzeichnet**, daß $R_1$ und $R_2$zusammen mit dem Stickstoffatom, das sie trägt, eine Gruppe der allgemeinen Formel

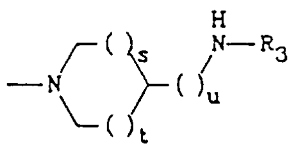

darstellen, in der s entweder die Zahl 0, und in diesem Fall t die Zahl 2, oder die Zahl 1, und in diesem Fall t die Zahl 1, u die Zahl 0 oder 1 und $R_3$ eine Gruppe der allgemeinen Formel

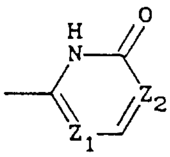

oder (Tautomer)

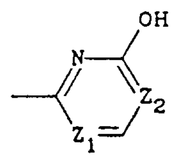

bedeuten, worin $Z_1$ urd $Z_2$ die in Anspruch 1 angegebenen Bedeutungen besitzen.

5. Verbindung nach Anspruch 1, **dadurch gekennzeichnet**, daß $R_1$ und $R_2$ zusammen mit dem Stickstoffatom, das sie trägt, eine Gruppe der allgemeinen Formel

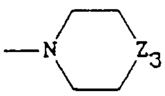

bilden, worin $Z_3$ eine Gruppe der allgemeinen Formel N-$R_4$ darstellt, in der $R_4$ ein Wasserstoffatom, eine Methylgruppe, eine Acetylgruppe, eine tert.-Butyloxycarbonylgruppe oder eine Phenyloxycarbonylgruppe bedeutet.

6. Verbindung nach Anspruch 1, **dadurch gekennzeichnet**, daß $R_2$ eine Gruppe der allgemeinen Formel

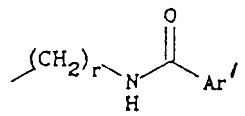

darstellt, in der r die Zahl 2 oder 3 und Ar' entweder eine Phenylgruppe, die gegebenenfalls durch eines oder mehrere Chloratome, Methoxygruppen oder Aminogruppen substituiert ist, oder eine Furan-2-yl-gruppe oder eine Tetrahydrofuran-2-yl-gruppe oder eine Pyridin-3-yl-gruppe bedeuten.

7. Verbindung nach Anspruch 1, **dadurch gekennzeichnet**, daß $R_2$ eine $C_2$-$C_3$-(Hydroxy)-(methoxy)-alkylgruppe oder eine $C_2$-$C_3$-Dimethoxyalkylgruppe bedeutet.

8. Verfahren zur Herstellung einer Verbindung nach Anspruch 1, **dadurch gekennzeichnet**, daß man ein Amid der allgemeinen Formel (II)

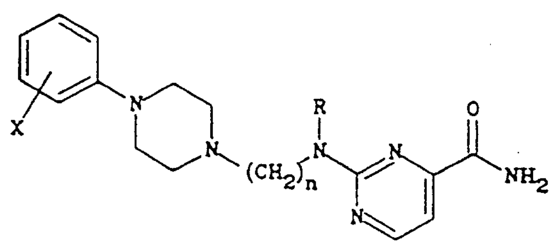

(II)

in der n, X und R die in Anspruch 1 angegebenen Bedeutungen besitzen, durch Umsetzen mit einem aliphatischen Alkohol in Gegenwart einer Base in einen Ester der allgemeinen Formel (III) umwandelt

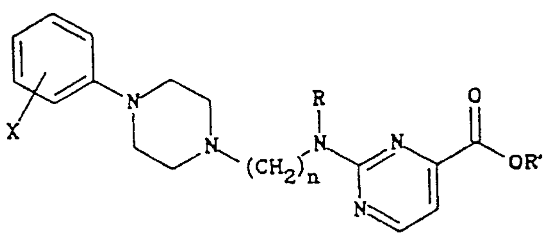

(III)

in der R' eine $C_1$-$C_4$-Alkylgruppe darstellt, und dann den in dieser Weise erhaltenen Ester mit einem Amin der allgemeinen Formel (IV)

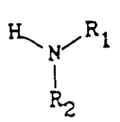

(IV)

in der $R_1$ und $R_2$ die in Anspruch 1 angegebenen Bedeutungen besitzen, direkt umsetzt, wenn das Amin ein primäres Amin ist ($R_1$ = H), oder nach vorausgehender Herstellung des entsprechenden Dimethylaluminiumamids mit Hilfe von Trimethylaluminium umsetzt, wenn dieses Amin ein sekundäres Amin ist ($R_1$ = $CH_3$).

9. Arzneimittel, **dadurch gekennzeichnet**, daß es aus einer Verbindung nach Anspruch 1 besteht.

10. Pharmazeutische Zubereitung, **dadurch gekennzeichnet**, daß sie eine Verbindung nach Anspruch 1 in Kombination mit einem pharmazeutischen Trägermaterial enthält.

**Patentanspruch für folgende Vertragsstaaten : ES, GR**

**1.** Verfahren zur Herstellung einer Verbindung der allgemeinen Formel (I)

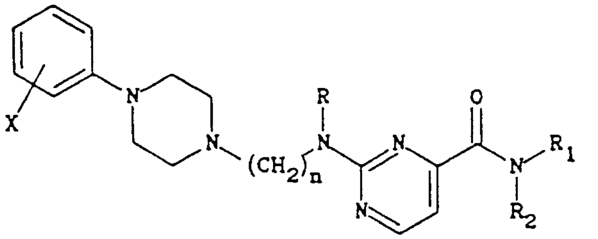

(I)

in der

n die Zahl 2 oder 3,

X eines oder mehrere Atome oder Gruppen ausgewählt aus: Wasserstoffatomen, Fluoratomen, Chloratomen und Methoxygruppen,

R ein Wasserstoffatom oder eine Methylgruppe,

$R_1$ ein Wasserstoffatom oder eine Methylgruppe,

$R_2$

- eine $C_1$-$C_6$-Alkylgruppe,
- eine $C_2$-$C_3$-Hydroxyalkylgruppe,
- eine $C_2$-$C_3$-(Hydroxy)-(methoxy)-alkylgruppe,
- eine $C_2$-$C_3$-Dimethoxyalkylgruppe,
- eine 2-(Aminosulfonyl)-ethylgruppe,
- eine 2-(Methylsulfonyl)-ethylgruppe,
- eine 2-(Methylsulfonylamino)-ethylgruppe,
- eine Gruppe der allgemeinen Formel $-CH_2-CO-NY_1Y_2$ (worin $Y_1$ und $Y_2$ unabhängig voneinander jeweils ein Wasserstoffatom oder eine $C_1$-$C_6$-Alkylgruppe darstellen),
- eine Gruppe der allgemeinen Formel $-(CH_2)_2-Ar$ (worin Ar eine Phenylgruppe, die gegebenenfalls durch eine oder mehrere Methoxy- oder Aminosulfonylgruppen substituiert ist, darstellt),
- eine Gruppe der allgemeinen Formel

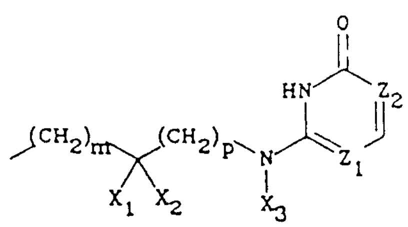

oder (Tautomer)

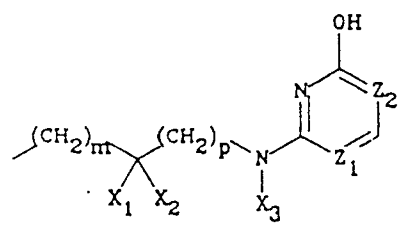

worin m die Zahl 0 oder 1, p die Zahl 1 oder 2, eines der Symbole $Z_1$ und $Z_2$ eine CH-Gruppe und das andere ein Stickstoffatom oder $X_1$, $X_2$ und $X_3$ jeweils ein Wasserstoffatom, oder $X_1$ und $X_2$ jeweils eine Methylgruppe und $X_3$ ein Wasserstoffatom, oder $X_1$ ein Wasserstoffatom und $X_2$ und $X_3$ gemeinsam eine Kette der allgemeinen Formel $-(CH_2)_{4-p}$ (worin p die oben angegebenen Bedeutungen besitzt) darstellen, oder

- eine Gruppe der allgemeinen Formel

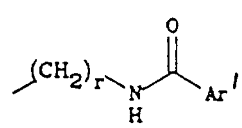

(in der r die Zahl 2 oder 3 und Ar' entweder eine Phenylgruppe, die gegebenenfalls

durch eines oder mehrere Chloratome, Methoxygruppen oder Aminogruppen substituiert ist, oder eine Furan-2-yl-gruppe oder eine Tetrahydrofuran-2-yl-gruppe oder eine Pyridin-3-yl-gruppe darstellt),

oder schließlich $R_1$ und $R_2$ gemeinsam mit dem Stickstoffatom, welches sie trägt,

- eine Gruppe der allgemeinen Formel

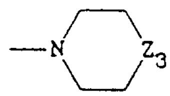

in der $Z_3$ ein Sauerstoffatom oder ein Schwefelatom, eine Sulfonylgruppe oder eine Gruppe der allgemeinen Formel N-$R_4$, worin $R_4$ ein Wasserstoffatom, eine Methylgruppe, eine Acetylgruppe, eine tert.-Butyloxycarbonylgruppe, eine Phenyloxycarbonylgruppe oder eine Gruppe der allgemeinen Formel

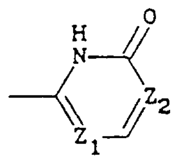 oder (Tautomer) 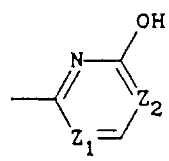

in der $Z_1$ und $Z_2$ die oben angegebenen Bedeutungen besitzen, darstellen, oder

- eine Gruppe der allgemeinen Formel

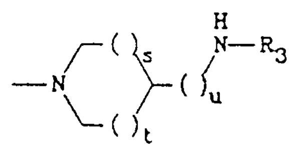

in der s entweder die Zahl 0, und in diesem Fall t die Zahl 2, oder die Zahl 1, und in diesem Fall t die Zahl 1, u die Zahl 0 oder 1 und $R_3$ ein Wasserstoffatom, eine tert.-Butyloxycarbonylgruppe, eine Phenyloxycarbonylgruppe oder eine Gruppe der allgemeinen Formel

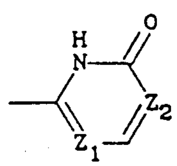 oder (Tautomer) 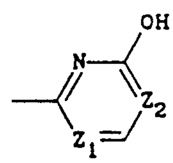

in der $Z_1$ und $Z_2$ die oben angegebenen Bedeutungen besitzen, bedeuten,

**dadurch gekennzeichnet**, daß man ein Amid der allgemeinen Formel (II)

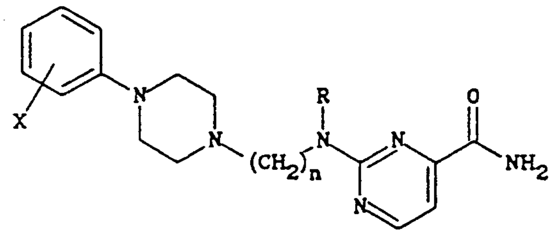

(II)

in der n, X und R die oben angegebenen Bedeutungen besitzen, durch Umsetzen mit einem

aliphatischen Alkohol in Gegenwart einer Base in einen Ester der allgemeinen Formel (III) umwandelt

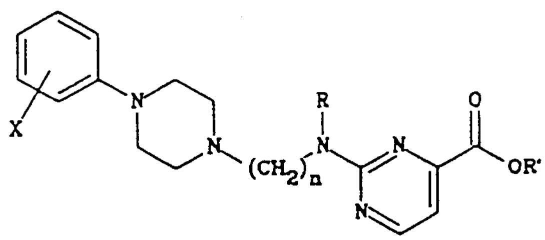

(III)

in der R' eine $C_1$-$C_4$-Alkylgruppe darstellt, und dann den in dieser Weise erhaltenen Ester mit einem Amin der allgemeinen Formel (IV)

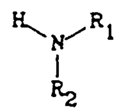

(IV)

in der $R_1$ und $R_2$ die in oben angegebenen Bedeutungen besitzen, direkt umsetzt, wenn das Amin ein primäres Amin ist ($R_1$ = H), oder nach vorausgehender Herstellung des entsprechenden Dimethylaluminiumamids mit Hilfe von Trimethylaluminium umsetzt, wenn dieses Amin ein sekundäres Amin ist ($R_1$ = $CH_3$).